# EUROPÄISCHE PATENTSCHRIFT

(11) **EP 1 268 566 B1**
(45) Veröffentlichungstag und Bekanntmachung des Hinweises auf die Patenterteilung: **16.06.2004**
(21) Anmeldenummer: 01921231.5
(22) Anmeldetag: 15.03.2001
(51) Int. Cl.: C08F 2/44, C08F 8/00

(54) **PORÖSE POLYMER/TRÄGER-FESTSTOFFPHASENREAKTANDEN, VERFAHREN ZU DEREN HERSTELLUNG UND DEREN VERWENDUNG**
POROUS POLYMER/CARRIER SOLID PHASE REACTANTS, METHOD FOR PRODUCING SAME AND THE USE THEREOF
REACTANTS POREUX EN PHASE SOLIDE POLYMERE/SUPPORT, LEUR PROCEDE DE PRODUCTION ET LEUR UTILISATION

(30) Priorität: 15.03.2000 DE 10012615
(43) Veröffentlichungstag der Anmeldung: 02.01.2003
(73) Patentinhaber: Kunz, Ulrich, 37520 Osterode (DE); Kirschning, Andreas, 38678 Clausthal-Zellerfeld (DE); Hoffmann, Ulrich, 37154 Northeim (DE)
(72) Erfinder: Kunz, Ulrich, 37520 Osterode (DE); Kirschning, Andreas, 38678 Clausthal-Zellerfeld (DE); Hoffmann, Ulrich, 37154 Northeim (DE)
(74) Vertreter: Schubert, Klemens, Dr.
(86) Internationale Anmeldenummer: PCT/DE2001/001092
(87) Internationale Veröffentlichungsnummer: WO 2001/068743

(56) Entgegenhaltungen:
- EP-A- 0 076 158
- EP-A- 0 416 369
- WO-A-00/75195
- GB-A- 1 450 193
- US-A- 4 130 512
- US-A- 4 842 936
- US-A- 5 653 922

## Beschreibung

Die Erfindung betrifft poröse Polymer/Träger-Feststoffphasenreaktanden, Verfahren zu deren Herstellung und deren Verwendung.

Die Synthese organisch-chemischer Produkte, bei denen die Reaktanden in Form von Lösungen eingesetzt werden, ist die klassische Arbeitsweise der organischen Chemie und schon lange Stand der Technik. Ein Nachteil ist die Handhabung größerer Lösemittelmengen und die damit verbundene schwierige Abtrennung gelöster erzeugter Produkte aus dem Reaktionsgemisch.

Eine Verbesserung stellt der Einsatz von Polymeren als Verankerungsmaterial für organische Reaktanden dar. Von Vorteil ist, dass die erzeugten Stoffe am festen Polymer gebunden bleiben und so gut von nicht umgesetzten Reaktanden und Nebenprodukten getrennt werden können.

Ein weithin bekanntes Beispiel ist die Synthese von Peptiden nach Merrifield (R. B. Merrifield: "Solid Phase Peptide Synthesis", J. Amer. Chem. Soc., Vol. 85, 2149-2154 (1963)). Umständlich ist die Handhabung des Polymers, welches als Kügelchen mit Abmessungen im Millimeterbereich vorliegt und nach Reaktionsende abfiltriert werden muss. Die eingesetzten Polymerkügelchen sind relativ groß, was aber den Stofftransport der Reaktanden zu den aktiven Stellen des Polymers verschlechtert. Dadurch werden die Reaktionszeiten lang.

Zur Vermeidung dieses Nachteils wäre der Einsatz von pulverförmigen Polymeren günstiger, dadurch verschlechtert sich aber die Handhabung und feine Polymerteilchen können die Produkte verunreinigen. Ein weiterer Nachteil der Merrifield-Synthese ist die notwendige Abtrennung des aufgebauten Produktes vom Polymer. Dies stellt einen zusätzlichen präparativen Schritt dar. Zusätzlich kann durch die erforderliche Abspaltungsreaktion auch gewünschtes Produkt angegriffen werden. Erschwerend kommt hinzu, dass die analytische Verfolgung der Reaktion schwierig ist, da die synthetisierten Verbindungen während der Synthese am Polymer gebunden bleiben.

Die ungeordnete Anordnung der Polymerphase als frei bewegliche Teilchen im Reaktionsmedium verhindert eine kontrollierte Anströmung und kann zu schlechten Ausbeuten und Selektivitäten beitragen.

Ein weiterer Nachteil handelsüblicher Materialien ist deren Beeinflussbarkeit durch unterschiedlich quellend wirkende Lösemittel. Dadurch werden Volumenveränderungen des quellenden oder schrumpfenden Polymers bewirkt. Dadurch verändern sich die Anströmbedingungen und der Stofftransport der Reaktanden zu den polymergebundenen Reaktanden in der Feststoffphase.

Die Verwendung von Polymeren zur Verankerung organischer Reaktanden und deren Umsetzung zu gelösten Produkten ist ebenfalls Stand der Technik (D. H. Drewry, D. Coe, S. Poon: Med. Res. Rev. (1999), 19, 97-148). Diese Synthesen erfolgen an Polymerkügelchen, die ca. 0,1 bis 1,5 mm Durchmesser aufweisen. Aufwendig ist auch hier die Abtrennung der Polymerphase von den Reaktanden und Produkten. Von Nachteil ist die schwierige Automatisierbarkeit, die die robotergesteuerte Durchführung der Arbeitsschritte Abwiegen der Polymerreaktanden, Dosieren von Feststoffen und Flüssigkeiten, Filtrieren und Trennen der Reaktionsprodukte verlangt.

Auf Grund der großen Polymerteilchen in nach dem Stand der Technik durchgeführten organischen Reaktionen wird nur ein Teil des Polymers in der gewünschten Reaktion nutzbar, die großen Teilchen behindern eine schnelle Diffusion der Reaktanden an die gebundenen organischen Reaktanden im Inneren der Polymerkügelchen. In der Regel muss mit großem (teuren) Überschuss an Reaktanden gearbeitet werden, um diesen Nachteil auszugleichen.

Von Vorteil wäre ein Material, welches die günstigen Eigenschaften fester, polymergebundener Reaktanden mit den Vorzügen der organischen Lösungschemie verbindet und darüber hinaus große Poren für eine konvektive Durchströmung besitzt.

Ein solcher Feststoffphasenreaktand sollte einerseits feine Teilchen besitzen, um einen guten Stofftransport und damit eine gute Ausnutzung in der Synthese zu gewährleisten und gleichzeitig möglichst große Abmessungen aufweisen, um die Handhabung zu vereinfachen. Wünschenswert wäre ein Feststoffphasenreaktand, der für verbesserte Methoden des Hochdurchsatz-Screening durch automatisierte Parallelsynthese geeignet wäre. Mit solch einem Material wäre die robotergesteuerte schnelle Generierung von Substanzbibliotheken potentieller neuer Wirkstoffe möglich.

Aufgabe der Erfindung ist es deshalb einen Polymer/Träger-Feststoffphasenreaktanden zur Verfügung zu stellen, der die Nachteile des Standes der Technik überwindet.

Überraschenderweise lässt sich mit erfindungsgemäßen, porösen Polymer/Träger-Feststoffphasenreaktanden diese Aufgabe lösen.

Die Aufgabe wird durch Schaffung eines porösen Polymer/Träger-Feststoffphasenreaktanden gelöst, wobei im Porenraum eines porösen Trägermaterials mit Porendurchmessern von 0,1 bis 2000 µm ein feinteiliges Polymer aus untereinander verbundenen Teilchen mit Durchmessern von 0,001 bis 50 µm vorliegt, an welchem organische Reaktanden gebunden sind.

Weiterhin bevorzugt ist es, dass der Porendurchmesser des porösen Trägermaterials 10 bis 500 µm beträgt.

Insbesondere bevorzugt ist es, dass der Teilchendurchmesser 1 bis 10 µm beträgt.

Vorteilhaft ist es, dass diese die Form von geordneten Packungen, Schüttgütern, Rohren, Platten und Stäben besitzen.

Weiterhin vorteilhaft ist es, dass diese Komponenten von Mikrotitrierfeldern sind.

Insbesondere vorteilhaft ist es, dass die Schüttgüter Kugeln, Raschig-Ringe oder Sattelkörper sind.

Besonders vorteilhaft ist es, dass als Polymere vernetzte Polystyrole, Polyhalogenstyrole, Polyacrylsäuren, Polyacrylsäureester, Polyacrylamide, Polyacrylnitrile, Polyvinylpyridine, Polyvinylcarbazole, Polyvinylbenzylchloride, Polyvinylaniline, Polyvinylbenzaldehyde, Poly-N-Vinylcaprolactame, Poly-4-Vinyl-1-cyclohexen-1,2-epoxide, Poly-3-Dimethylaminoacrylonitrile und Poly-N,N-Dimethylvinylbenzylamine entweder allein oder als Copolymere in den porösen Trägermaterialien vorhanden sind.

Bevorzugt ist es, dass als Polyhalogenstyrole Poly-4-Bromstyrol, Poly-2-Bromstyrol, Polyvinylbenzylbromid und Polyvinylbenzylchlorid verwendet werden.

Weiterhin bevorzugt ist es, dass die Polymere mit Divinylbenzol vernetzt sind.

Bevorzugt ist in jedem Fall, dass der Vernetzungsgrad 2 bis 55 % beträgt.

Insbesondere bevorzugt ist es, dass die eingelagerten Polymere Ankergruppen für organische Reaktanden besitzen, wie Aminogruppen, quaternäre Aminogruppen, Halogengruppen, Chlormethylgruppen, Sulfochloridgruppen, Sulfonsäuregruppen, Aldehydgruppen, Lactamgruppen, Epoxidgruppen, Hydroxygruppen, Carbonsäuregruppen und Carbonsäurehalogenidgruppen.

Vorteilhaft ist es, dass die in den Tabelle 1 bis 7 genannten organische Reaktanden tragenden Polymere im Porenraum der Trägermaterialien vorliegen.

Besonders vorteilhaft ist es, dass als poröse Trägermaterialien Gläser, Keramiken, Glaskeramiken, Polymere, Metalle, Legierungen, Stoffe wie Gesteine, Koks oder Kohlen eingesetzt werden.

Insbesondere vorteilhaft ist es, dass als Gesteine Bimsstein und Silikate verwendet werden.

Ein weiterer Gegenstand der vorliegenden Erfindung ist ein Verfahren zur Herstellung von erfindungsgemäßen Polymer/Träger-Feststoffphasenreaktanden, wobei man ein vernetztes Polymer in das Trägermaterial einlagert und dieses Polymer durch eine nachfolgende Behandlung mit weiteren Monomeren funktionalisiert, wobei das Polymer gegebenenfalls Ankergruppen besitzt.

Dabei ist es erfindungsgemäß bevorzugt, dass man die weiteren Monomere durch Eindiffundieren in die Polymerteilchen einbringt diese weiterhin mit einem Vernetzer umsetzt.

Weiterhin ist erfindungsgemäß bevorzugt, dass man an die Ankergruppen an sich bekannte organische Reaktanden bindet. Erfindungsgemäß besonders bevorzugt sind die in den Tabellen 1 bis 7 dargestellten Reaktanden.

Ein weiterer Gegenstand der vorliegenden Erfindung ist die Verwendung eines erfindungsgemäßen Polymer/Träger-Feststoffphasenreaktanden zur Durchführung von Additionsreaktionen, Oxidationen, Reduktionen, Epoxidierungen, Halogenierungen, Kohlenstoff-Kohlenstoff-Kupplungsreaktionen, nukleophilen Substitutionen, Dehydrierungen, Veresterungen, Veretherungen, Acylierungen, Ringöffnungen, Cyclisierungen, Isomerisierungen, elektrophilen Substitutionen, sowie von Reinigungsschritten und Mehrstufenreaktionen mittels der vorhandenen organischen Reaktanden.

Besonders bevorzugt ist dabei, dass es sich bei den Reinigungsreaktionen um "resin capture-and-release" und "scavenger" handelt.

Insbesondere ist dabei erfindungsgemäß bevorzugt, dass Stoffsynthese und Trennung im Polymer/Träger-Feststoffphasenreaktanden kombiniert sind.

Der Einsatz der erfindungsgemäßen Feststoffphasenreaktanden erlaubt neue Technologien für Herstellung neuer Substanzen und deren Screening auf Wirksamkeit. Durch die Bereitstellung der innovativen Polymer/Träger-Feststoffphasenreaktanden mit guter Zugänglichkeit der reaktiven Stellen durch konvektive Durchströmung wird der Stofftransport verbessert und die Ausnutzung der Polymerphase gesteigert. Die Porengröße im Mikrometerbereich erlaubt größere Geschwindigkeit beim Spülen und Versorgen mit neuen Reaktionspartnern. Dadurch steht eine hocheffiziente Synthesemethode zur Darstellung neuer potentieller Wirkstoffe zur Verfügung.

Die hier beschriebenen erfindungsgemäßen Polymer/Träger-Feststoffphasenreaktanden lassen sich in Form von regellosen Schüttungen, geordneten Packungen, als Rohre, Platten oder Stäbe herstellen und verwenden. Von besonderem Vorteil ist der Einsatz in Form von Stäben oder Platten, welche druckdicht in ein Gehäuse eingebaut werden und deren Einsatz in Syntheserobotern. Durch den Einsatz von Robotertechnologie und den Polymer/Träger-Feststoffphasenreaktanden steht eine Methode zur Verfügung, die es erlaubt ausreichend Nachschub an potentiellen Wirkstoffen sowie neue Targets für Testsubstanzen zu erzeugen. In Kombination mit Methoden der chromatographischen Trenntechnik ist die Erzeugung von Wirkstoffbibliotheken mit reinen Substanzen in kurzer Zeit möglich.
Durch die kleinen Polymerteilchen und die gleichzeitig vorhandenen großen Poren lassen sich auch die Zeiten für die Regenerierung der Feststoffphasenreaktanden erheblich verkürzen bzw. erst wirtschaftlich durchführen.

Die Vorteile der erfindungsgemäßen Polymer/Träger-Feststoffphasenreaktanden sind insbesondere:
- Nach der Erzeugung der aktiven Polymerphase im Porenraum der Trägermaterialien ist das Polymer immobilisiert. Es bildet eine zusammenhängende Phase, die bei konvektiver Durchströmung den Porenraum nicht verlassen kann.
- Die Beladung mit Polymer wird durch einen gut zu kontrollierenden Polymerisationsprozess erzeugt. Dadurch ist eine gleichmäßige Beladung des Porenvolumens der Trägermaterialien mit aktiver Polymerphase sichergestellt.
- Die Beladung mit Polymer ist durch die Wahl der Konzentration der Monomeren während des Polymerisationsprozesses möglich. Dadurch ist eine Optimierung hinsichtlich Kapazität oder Reaktionszeit möglich.
- Übliche nach dem Stand der Technik bekannte Verfahren zur Aktivierung der Polymerphase sind leicht durchführbar. Dadurch ist eine Anpassung an verschiedene Syntheseaufgaben möglich.
- Das Polymer bildet eine zusammenhängende Phase. Diese bleibt durch die Einbettung in das Trägermaterial formstabil, auch bei starken Änderungen der Solvatisierungseigenschaften der verwendeten Lösemittel.
- Durch die Verwendung eines porösen Trägers mit großen Poren zur Einlagerung für das die reaktiven Gruppen tragende Polymer und die chemische Verbindung der Polymerteilchen untereinander sind sowohl kleine Polymerteilchen wie gleichzeitig auch große Poren zu verwirklichen. Dadurch ergeben sich beim Einsatz in Durchflußapparaturen geringe Druckverluste und hohe Durchsätze an Reaktanden sind möglich.
- Ein Polymer/Träger-Feststoffphasenreaktand in stückiger Form oder als Stab oder Platte ist als Reaktand in organischen Synthesen leichter zu handhaben als ein feinteiliges Pulver.
- Durch den formstabilen Träger in Kombination mit einer formschlüssigen Gehäuseausführung kann die Randgängigkeit eines flüssigen oder gelösten Reaktanden bei Durchströmung durch den Feststoffphasenreaktanden ausgeschlossen werden.
- Durch den Einsatz der beschriebenen Feststoffphasenreaktanden ist die Aufarbeitung der Reaktionsprodukte wesentlich einfacher.
- Die leichte Regenerierbarkeit der Materialien, besonders in Durchflußapparaturen, verkürzt die Produktionszyklen.
- Die Steigerung der Sicherheit bei der Handhabung durch die Bindung der Reaktanden an die feste Polymerphase vermeidet den Umgang mit niedrig siedenden Gefahrstoffen.
- Die gesteigerte Reaktivität durch die Anbindung der Reaktanden an eine disperse Feststoffmatrix erhöht die Produktivität.

Die nachfolgenden Beispiele erläutern die Erfindung:

### I. Herstellung von porösen Polymer/Träger-Feststoffphasenreaktanden

### I.1. Einlagerung von vernetzten polymeren in den Porenraum von Trägermaterialien

### a) Polymerisation aus Vinylbenzylchlorid und Divinylbenzol

Die Herstellung des erfindungsgemäßen porösen Polymer/Träger-Feststoffphasenreaktanden wird anhand der Polymerisation von Vinylbenzylchlorid mit Divinylbenzol beschrieben.

Für die Einlagerung von vernetzten Polymeren in den Porenraum von Trägermaterialien wird eine Mischung aus Vinylbenzylchlorid und Divinylbenzol in einem Lösungsmittel, welches die Monomeren gut löst, das Polymer aber schlecht löst, hergestellt. Nach Zugabe eines Initiators, z.B. Azoisobutyronitril, wird nach dessen Auflösung ein oder mehrere poröse Trägermaterialstücke, z.B. Stäbe oder Platten aus porösem Sinterglas, in die Mischung gegeben, so dass sie vollständig mit der Reaktionsmischung überdeckt sind. Die Konzentration der Monomeren kann zur Einstellung der Polymerbeladung der Träger variiert werden. Dann wird kurz Vakuum angelegt, um Luftblasen aus dem porösen Träger zu entfernen. Durch Erwärmung beginnt die Polymerisation, welche durch die schlechte Löslichkeit der gebildeten Polymerphase zur Bildung eines Feststoffes im Porenraum der Trägermaterialien führt. Nach Abschluss der Polymerisation wird der Träger aus der Reaktionslösung entfernt und von anhaftenden Polymerresten gereinigt.

Zur weiteren Steigerung der Polymerbeladung ist es möglich, das beschriebene Polymerisationsverfahren mehrmals durchzuführen.

Ergebnis dieses Herstellungsverfahrens ist eine aus verbundenen Teilchen bestehende Polymerphase aus vernetztem Polyvinylbenzylchlorid im Porenraum des Trägermaterials. Die Chlormethylgruppe kann für die Ankopplung anderer reaktiver Gruppen genutzt werden.

### b) Polymerisation anderer Monomere/Copolymere mit Divinylbenzol

Auf ähnliche Art und Weise ist auch die Polymerisation von anderer Monomeren/Copolymeren mit Divinylbenzol möglich.

So lassen sich Aminostyrole, Styrol, Halogenstyrole, Vinylbenzaldehyde, Vinylcarbazole, N-Vinylcaprolactame, 4-Vinyl-1-cyclohexen-1,2-epoxide, Vinylpyridine, 3-Dimethylaminoacrylonitrile, N,N-Dimethylvinylbenzylamine sowie Acrylonitril in den Porenraum von Trägermaterialien einlagern.

Auch die Herstellung von Copolymeren ist möglich. Dabei ist es erforderlich die Art und Menge des verwendeten Lösungsmittels/Fällungsmittels auf die Lösungseigenschaften der Monomeren und Polymere abzustimmen.

Andere geeignete Lösemittel/Fällungsmittel sind Alkohole wie Methanol, Ethanol, Propanole und Butanole sowie Kohlenwasserstoffe, in einigen Fällen auch Wasser. Dabei können die genannten Lösemittel einzeln oder auch in Mischungen zur Herstellung von porösen Polymer/Träger-Feststoffphasenreaktanden eingesetzt werden. Bei schwer löslichen Monomeren können der Reaktionsmischung solche Stoffe zugesetzt werden, die die Löslichkeit für die Monomeren erhöhen wie z.B. Aromaten oder andere geeignete Stoffe.

Bei Polymeren, die sich durch eine Fällungspolymerisation nicht oder nur unzureichend in die Träger einlagern lassen, wird zunächst ein vernetztes Polystyrol oder ein anderes gut einlagerbares Polymer in die Träger wie oben beschrieben eingebracht. Dann wird dieses Produkt mit einer Lösung des zusätzlichen Polymers in einem das bereits vorhandenen Polymer aufquellenden Lösemittel behandelt. Das zusätzliche Monomer gelangt in die Gelphase des bereits vorhandenen Polymers und kann mit bekannten Vernetzungsmethoden in diesem gebunden werden.

Durch die beschriebenen Methoden stehen Herstellungsverfahren für poröse Polymer/Träger-Feststoffphasenreaktanden zur Verfügung, die es erlauben, eine breite Palette an Ankerpolymeren für organische Reagenzien im Porenraum von Trägern zu fixieren.

Im Folgenden werden Ausführungsbeispiele der erfindungsgemäßen Polymer/Träger-Feststoffphasenreaktanden gegeben, wobei sich die verwendete Nummerierung der Verbindungen auf die Nummern der Strukturen bezieht, die in den Tabellen 1 bis 7 aufgeführt sind.

### I.2. Aktivierung von Polymeren mit Ankergruppen für organische Reaktanden im Porenraum von Trägermaterialien

Die folgenden Ausführungsbeispiele beschreiben die Aktivierung von Polymeren mit Ankergruppen für organische Reaktanden im Porenraum von Trägermaterialien.

### a) Aminierung von vernetztem Polyvinylbenzylchlorid

200 ml, mit vernetztem Polyvinylbenzylchlorid belegte Träger, werden in einem 1 Liter 3-Hals-Kolben mit 350 ml wasserfreiem Toluol versetzt. Der Kolben wird mit Thermometer, Rückflusskühler, Gaseinleitrohr, Gasableitrohr und Trockenrohr versehen. Es wird auf 50 bis 60 °C temperiert. Dann wird langsam wasserfreies Trimethylamin eingeleitet. Dieses wird durch Eintropfen von 45 %iger wässriger Trimethylaminlösung in eine konzentrierte Natronlauge und anschließende Trocknung des Gasstromes über festem Natriumhydroxid hergestellt. Das Begasen wird für ca. 5 Stunden fortgesetzt, wobei die Flüssigkeit über den Trägern leicht gerührt wird. Die Reaktionsmischung wird über Nacht stehengelassen und der Vorgang des Begasens am nächsten Tag wiederholt. Es wird nochmals über Nacht stehengelassen. Dann wird die Lösung dekantiert und durch neues wasserfreies Toluol ersetzt. Man lässt wieder über Nacht stehen. Dann werden die Träger aus der Lösung entfernt und bei 65 °C im Vakuum getrocknet. Die Träger werden über Nacht mit 5 %iger Salzsäure behandelt und anschließend mit Wasser säurefrei gewaschen. Ergebnis dieser Prozedur ist ein quarternäre Aminogruppen enthaltendes feinteiliges Polymer im Porenraum des Trägermaterials (Chloridform der Verbindung Nr. 1).

### b) Sulfochlorierung von vernetztem Polystyrol

100 ml Chloroform werden mit 50 ml Chlorsulfonsäure gemischt. Bei Raumtemperatur werden 100 ml getrocknete, mit vernetztem Polystyrol beladene Trägermaterialien hinzugefügt. Für 24 Stunden wird die Mischung unter Feuchtigkeitsausschluß bei Raumtemperatur gehalten. Nach Beendigung der Umsetzung mit Chlorsulfonsäure wird die Chlorsulfonsäurelösung abgegossen und die polymergefüllten Trägermaterialien werden mehrfach mit Chloroform gespült und im Vakuum getrocknet. Die Zusammensetzung des Sulfochlorierungsgemisches und die eingesetzte Menge kann je nach gewünschtem Grad der Sulfochlorierung angepaßt werden.
Ergebnis dieses Syntheseschrittes sind polymergebundene Sulfochloride im Porenraum poröser Trägermaterialien (Verbindung Nr. 67).

### c) Methylierung von vernetztem Polyvinylpyridin

5 g vernetztes Polyvinylpyridin enthaltender Träger werden mit der 2-fachen molaren Menge an Methyliodid zur Reaktion gebracht. Anstelle von Methyliodid ist auch Dimethylsulfat geeignet. Durch diese Reaktion gelingt die Herstellung eines basischen Feststoffphasenreaktanden, welcher für weitere Synthesen verwendet werden kann. Durch die Verwendung von Polyvinylpyridin als eingelagertes feinteiliges Polymer kann die Aminierung mit Trimethylamin vermieden werden.

### I.3. Austausch der Chloridgruppe von Polymer/Trägermaterialien gegen andere reaktive Ionen

### a) Austausch gegen Iodid

Das unter Ausführungsbeispiel I.2.a) erhaltenen Material (Chloridform der Verbindung Nr. 1) wird mit konzentrierter (57%ig) Iodwasserstoffsäure behandelt. Alternativ kann im Hydrolyseschritt I.2.a) direkt konzentrierte Iodwasserstoffsäure zugesetzt werden. Die als Gegenion zu den quarternären Aminofunktionen fungierenden Chloridionen werden durch diesen Schritt gegen Iodionen ausgetauscht. Mit der Einführung des Iodidions in das Polymer steht ein hochreaktiver poröser Polymer/Träger-Feststoffphasenreaktand für organische Synthesen zur Verfügung (Verbindung Nr. 72).

### b) Austausch von Chloridionen gegen andere reaktive Ionen

Neben dem oben beschriebenen Beispiel ist auch der Austausch von Chloridionen gegen andere reaktive Anionen möglich.
Die folgende Auflistung nennt einige Beispiele:
Borhydridionen(Verbindung Nr. 39), Cyanoborhydrid (Verbindung Nr. 40), Azidionen (Verbindung Nr. 73), Cyanidionen (Verbindung Nr. 71), Rhodanidionen (Verbindung Nr. 76), Isocyanationen (Verbindung Nr. 83), Perjodationen (Verbindung Nr. 38), Permanganationen (Verbindung Nr. 17) sowie die anderen in den Tabellen 1 bis 7 genannten Anionen.

### I.4. Austausch der Iodidgruppe gegen Diacetoxyiodat-(I)-Anion

Durch Umsetzung des unter I.3.a) erhaltenen Materials (Verbindung Nr. 72) mit Diacetoxyiodbenzol (Iodosobenzoldiacetat) wird Diacetoxyiodat in das Polymer eingeführt. Durch diesen Syntheseschritt entsteht ein Diacetoxyiodat enthaltender Polymer/Träger-Feststoffphasenreaktand (Verbindung Nr. 60).

Ergebnis dieser Herstellungsverfahren ist ein mit einer aus untereinander verbundenen kugelförmigen Teilchen bestehenden Polymerphase gefüllter Träger, welcher gebundene reaktive Gruppen enthält, die in organisch-chemischen Synthesen als Reaktanden eingesetzt werden können. Eine Auswahl zugänglicher Polymer/Träger-Feststoffphasenreaktanden sowie der mit diesen möglichen organischen Reaktionen ist in den Tabelle 1 bis 7 zu finden. Anionen und Kationen der genannten Verbindungen können in einigen Fällen durch gegenseitigen Tausch zu neuen Feststoffphasenreaktanden verbunden werden. Dadurch vergrößert sich die Anzahl an zugänglichen Polymer/Träger-Feststoffphasenreaktanden erheblich.

### II. Beispiele für die Verwendung poröser Polymer/Träger-Feststoffphasenreaktanden in organisch-chemischen Synthesen

Um die Wirksamkeit der hier beschriebenen Polymer/Träger-Feststoffphasenreaktanden zu demonstrieren, wurden zunächst Untersuchungen durchgeführt, bei denen die Feststoffphasenreaktanden mit Alkenen umgesetzt wurden.

### II.1. Umsetzung eines porösen Polymer/Träger-Feststoffphasenreaktanden mit einem Olefin bei freier konvektiver Umströmung

### a) Umsetzung von polymergebundenem Diacetoxyjodat mit Anethol

2 g poröser Polymer/Träger-Feststoffphasenreaktand in Form von Raschig-Ringen, erhalten nach Beispiel I.4 (enthält im Trägerporenraum Verbindung Nr. 60) wird mit einer Lösung von 1 mmol Anethol (1-Methoxy-4-propenylbenzol) in wasserfreiem Dichlormethan bei Raumtemperatur zur Reaktion gebracht. Dabei werden Iod und eine Acetylgruppe an die Doppelbindung des Alkens angelagert. Die Reaktion ist nachfolgend dargestellt.

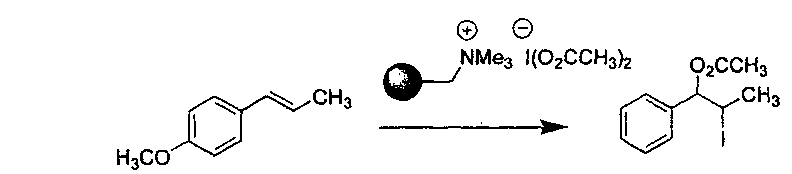

Überraschenderweise zeigte sich bei diesem Experiment, dass durch die Verwendung der hier beschriebenen porösen Polymer/Träger-Feststoffphasenreaktanden bereits in der Anwendungsform von frei in der Lösung umströmten Trägern eine bessere Ausnutzung der Polymerphase im Vergleich zu kommerziellen Polymeren erzielt werden konnte. Während bei den kommerziellen Harzen, welche Teilchendurchmesser bis zu einigen Millimetern aufweisen, mit einem 300 %igen Überschuss an Reaktanden gearbeitet werden musste, reichte bei den erfindungsgemäßen Materialien ein Überschuss von nur 50 % aus. Gleichzeitig ließ sich die Dauer der Synthese erheblich verkürzen.

Der Einsatz der erfindungsgemäßen Materialien in Form von konvektiv durchströmbaren Bauteilen in organisch-chemischen Synthesen wird im Folgenden beschrieben. Es werden Experimente beschrieben, bei denen verschiedene Feststoffphasenreaktanden hergestellt und verwendet wurden.

### II.2. Beispiel für die Verwendung von porösen Polymer/Träger-Feststoffphasenreaktanden in Form eines konvektiv durchströmbaren Bauteils

### II.2.1. Herstellung eines porösen Polymer/Träger-Feststoffphasenreaktanden mit Borhydridionen

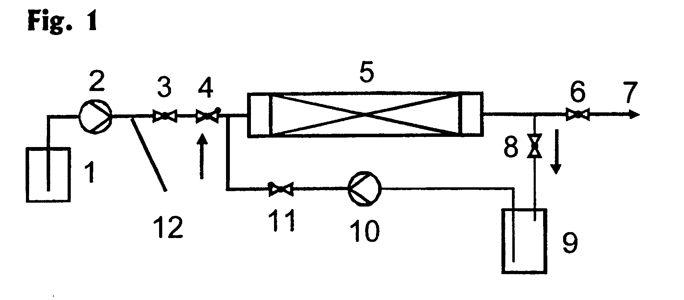

Hierzu wird zunächst ein Polymer/Träger-Feststoffphasenreaktand in Form eines Stabes von 5 mm Durchmesser und 10 cm Länge randgängigkeitsfrei in ein druckfestes Rohr eingebaut. Dieser Reaktand enthält als reaktive Gruppe die Chloridform der Verbindung Nr. 1. Durch diesen Stab wird bei Raumtemperatur eine 1 molare wässrige Lösung von Natriumborhydrid geleitet. Der Versuchsaufbau ist in Abbildung 1 schematisch dargestellt. Aus einem Vorratsbehälter 1 wird durch eine Pumpe 2 die genannte Lösung durch einen geöffneten Absperrhahn 3 und ein Rückschlagventil 4 durch den porösen Polymer/Träger-Feststoffphasenreaktanden 5 gefördert. Die Lösung verlässt nach Passieren des Regelventils 6 die Apparatur bei 7. Der Absperrhahn 8 ist bei diesem Versuch geschlossen, die Pumpe 10 nicht eingeschaltet. 9 ist ein Pufferbehälter, 11 ein Rückschlagventil zum Schutz der Pumpe.

Durch das Hindurchleiten von Natriumborhydridlösung durch den Chloridionen tragenden Stab erfolgt durch Ionenaustausch der Ersatz der Chloridionen durch Borhydridionen. Werden in der bei 7 austretenden Lösung keine Chloridionen mehr nachgewiesen, so wird die Dosierung der Natriumborhydridlösung beendet und Methanol in den Vorratsbehälter 1 gefüllt. Dann wird solange mit Methanol gespült, bis die die Apparatur verlassende Lösung kein Natriumborhydrid mehr enthält. Anschließend wird mit Dichlormethan oder Chloroform gespült und die Trocknung im Vakuum vervollständigt.

Ergebnis dieses Experimentes ist ein Borhydridionen tragender poröser Polymer/Träger-Feststoffphasenreaktand (Verbindung Nr. 39), welcher z.B. für Reduktionen eingesetzt werden kann.

### II.2.2. Reduktion von Acetophenon unter Verwendung eines Borhydridionen tragenden porösen Polymer/Träger-Feststoffphasenreaktanden

### Für dieses Experiment kommt dieselbe Apparatur, wie in Beispiel II.2.1. beschrieben, zum Einsatz.

Der getrocknete poröse Polymer/Träger-Feststoffphasenreaktand 5 (Verbindung Nr. 39) wird aus einem Vorratsbehälter 1 mit Methanol, Ethanol oder Propanolen bei Raumtemperatur durch die Pumpe 2, den geöffneten Absperrhahn 3 und das Rückschlagventil 4 versorgt. Durch Öffnen des Ventils 8 und Einschalten der Pumpe 10 wurde der Kreislaufstrom in Betrieb genommen. Nach Einstellung stabiler Strömungszustände mit Hilfe von Ventil 6 wird in den Behälter 9 eine Menge von 1 mmol Acetophenon gelöst in Alkohol mit Hilfe einer Spritze dosiert. Gleichzeitig wird der Absperrhahn 3 und Ventil 6 geschlossen und bei Raumtemperatur die das Acetophenon enthaltende Lösung im Kreislauf durch den Feststoffphasenreaktanden geleitet. In zeitlichen Abständen werden aus dem Behälter 9 Proben entnommen und die Konzentration des gebildeten Produktes Phenylethylalkohol bestimmt. Zur Entnahme des Produktes wird Ventil 6 geöffnet und Produkt enthaltende Lösung bei 7 der Apparatur entnommen. Hierzu wird Absperrhahn 3 wieder geöffnet und reines Lösemittel solange in die Apparatur gefördert, bis das in der Apparatur gebildete Produkt vollständig bei 7 entnommen wurde.

Die Reaktionsgleichung dieser Umsetzung lautet wie folgt:

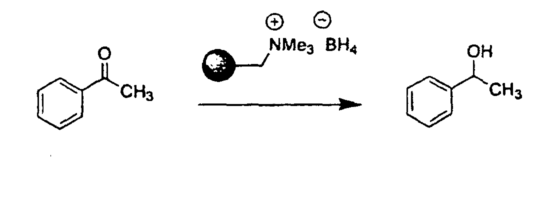

Bei diesem Ausführungsbeispiel zeigt sich besonders vorteilhaft die gute Durchströmbarkeit der hier beschriebenen porösen Polymer/Träger-Feststoffphasenreaktanden und die gute Handhabbarkeit, welche eine wesentlich einfachere Abtrennung und Aufarbeitung als bei handelsüblichen kugelförmigen Polymeren nach dem Stand der Technik erlaubt. Umständliches Filtrieren und chargenweises Waschen der handelsüblichen Harze werden vermieden.

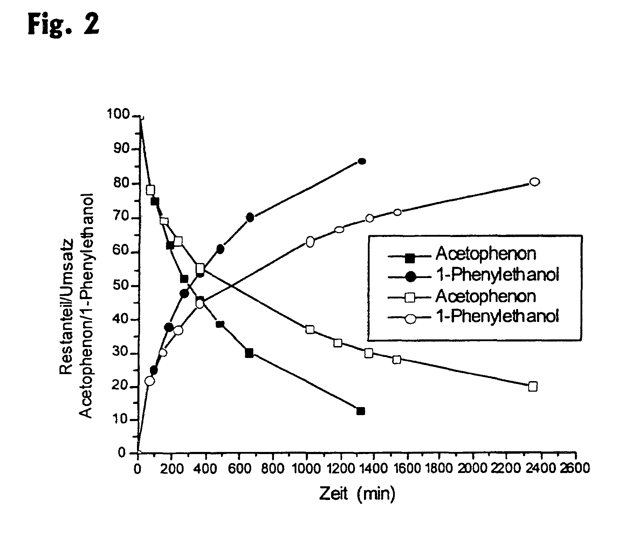

In Abbildung 2 sind die erhaltenen Versuchsergebnisse vergleichend den Resultaten eines Experiments, bei dem ein zerkleinerter Stab bei freier konvektiver Umströmung eingesetzt wurde, gegenübergestellt. Es zeigt sich deutlich, dass bei Einsatz des durchströmten stabförmigen Feststoffphasenreaktanden deutlich höhere Umsätze nach kürzeren Zeiten erhalten werden.

Mittels einer Vorrichtung gemäß Fig. 1 wurde der erfindungsgemäße Polymer/Träger-Feststoffphasenreaktand für verschiedene chemische Transformationen eingesetzt. Hierbei wurde darauf geachtet, zunächst ionisch gebundene Reagentien zu nutzen. Diese Vorgehensweise ließ es zu, auch die Wiederverwertbarkeit der Reaktoren für gleiche und für weitere chemische Transformationen zu bestimmen. Folgende Reaktionen ließen sich mit einem Mikroreaktor nacheinander realisieren. In der nachfolgenden Tabelle A sind weitere beispielhafte Reaktionen aufgeführt, welche mit den erfindungsgemäßen Polymer/Träger-Feststoffphasenreaktanden durchgeführt wurden. Es sind dies im Einzelnen:
1. Reduktion von Acetophenon zu 1-Phenylethanol mit immobilisierten Borhydridionen (Beispiel 1).
2. TEMPO-vermittelte Oxidation von Cyclohexanol zu Cyclohexanon und eines Steroidalkohols zu dem Keton mit dem immobilisertem Bromat-Komplex (Beispiele 2 und 3).
3. Desilylierung eines Steroids mit dem immobilisierten Fluoridion und Bildung des Alkohols (Beispiel 4)
4. Reduktive Aminierung des Ketons und Bildung des Benzylamins unter Nutzung des immobiliserten Borhydridions (Beispiel 5).
5. Substitutionsreaktionen an Benzylbromid mit immobilisierten Azid- und Cyanidionen (Beispiele 6 und 7).
6. Substitutionsreaktion an Oktyliodid mit dem immobilisierten Thiocyanation (Beispiel 8).

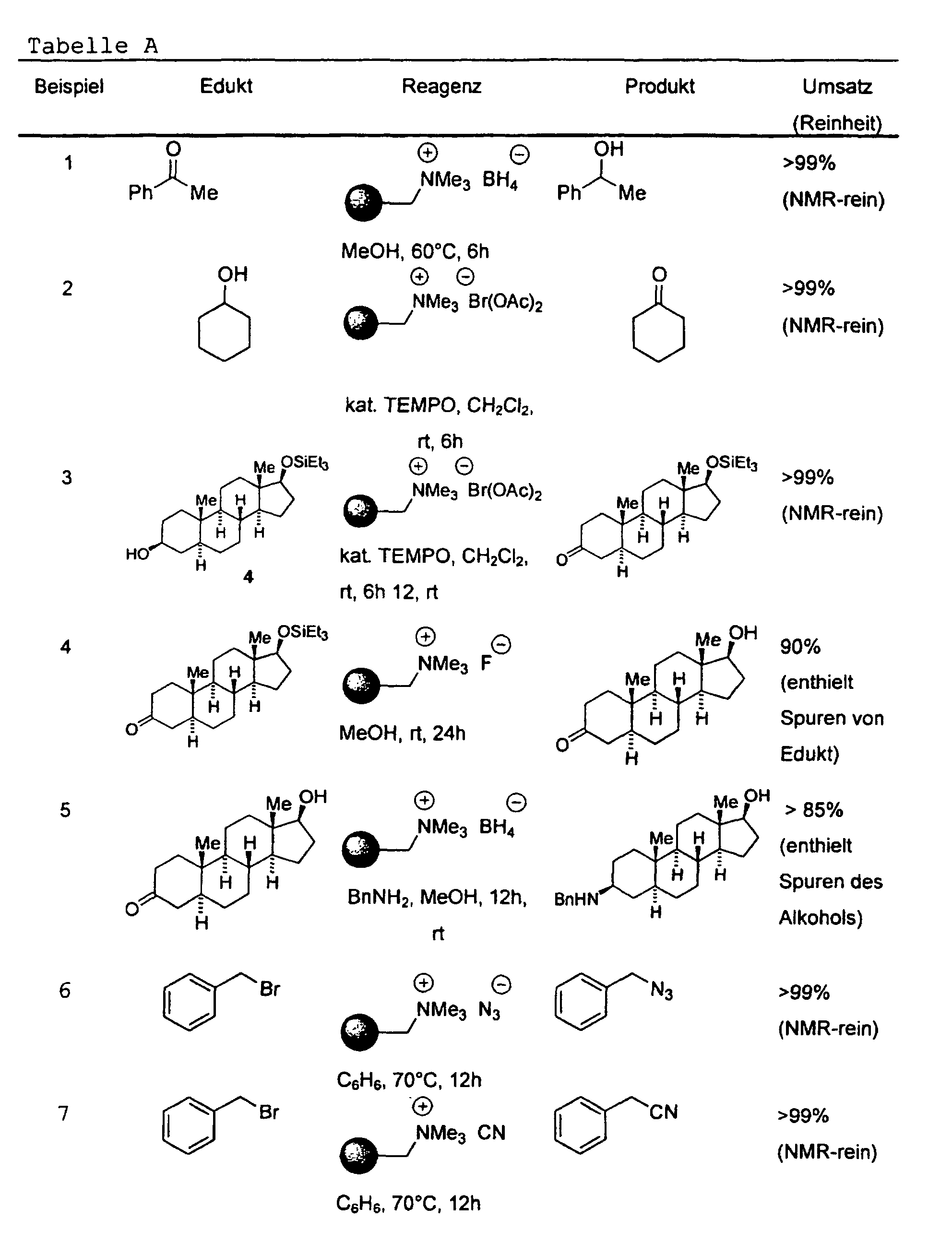

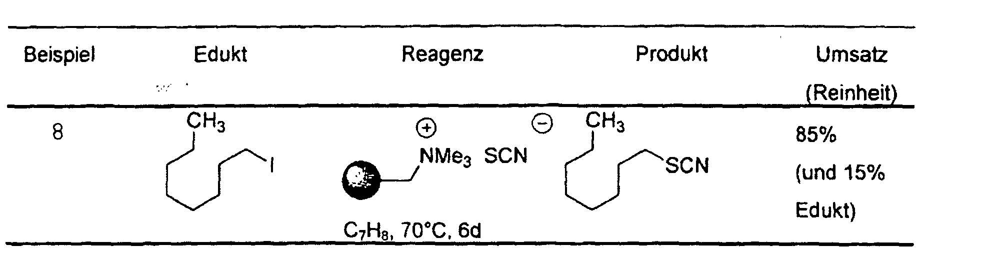

Nachfolgend werden einige weitere Reaktionen beschrieben, welche mit der erfindungsgemäßen Vorrichtung unter Verwendung der erfindungsgemäßen Polymer/Träger-Feststoffphasenreaktanden durhgeführt wurden.

### 1. Reduktion von Acetophenon (generelle Vorschrift für NaBH₄-Reduktion)

Die Chlorid-Form des Mikroreaktors wurde nacheinander behandelt mit NaOH (10 mmol in 10 mL Wasser), demineralisiertem Wasser (10 mL), Natriumborhydrid (1g in 10 mL Wasser), demineralisiertem Wasser (10 mL) und Methanol (10 mL). Eine Lösung von Acetophenon (0.5 mmol in 10 mL Methanol) wurde bei 60°C über 6h im Kreislauf durch den Reaktor geführt bis die Reaktion vollständig war. Der Reaktor wurde mit Methanol (10 mL) gespült und das Lösungsmittel im Vakuum entfernt. Ausbeute an 1-phenylethanol: > 99 %.

### 2. Oxidation von Cyclohexanol (generelle Vorschrift für die TEMPO-katalysierte Oxidation)

Die Chlorid-Form des Mikroreaktors wurde nacheinander behandelt mit NaOH (10 mmol in 10 mL Wasser), demineralisiertem Wasser (10 mL), HBr (2N, 10 mL), demineralisiertem Wasser (10 mL), wasserfreiem Methanol (10 mL) und wasserfreiem Dichlormethan. Anschließend wurde eien Lösung von Iodbenzol- Diacetat (1.5 mmol in 10 mL wasserfreiem Dichloromethan) 12h bei Raumtemperatur im Kreislauf durch den Reaktor gepumpt. Der Reaktor wurde gewaschen mit wasserfreiem Dichloromethan (10 mL). Eine Lösung von Cyclohexanol (0.125 mmol in 10 mL wasserfreiem Dichloromethan) mit einer katalytischen Menge TEMPO wurde unter Argon in der Säule zur Reaktion gebracht, bis der Umsatz vollständig war 86h). Der Reaktor wurde gespült mit Dichlormethan (10 mL) und das Lösungsmittel im Vakuum abgezogen. Ausbeute an Cyclohexanon: > 99 %).

### 3. Oxidation von Steroiden

In Analogie zu 2. Wurden Steroidalkohole zur Reaktion gebracht. Ausbeute an Steroidketonen: 99%.

### 4. Desilylierung (eines Steroides)

Die Chlorid-Form des Mikroreaktors wurde nacheinander behandelt mit NaOH (10 mmol in 10 mL Wasser), demineralisiertem Wasser (10 mL), HF (2N, 10 mL) und wasserfreiem Methanol (15 mL). Eine Lösung des O-Triethylsilyl- geschützten Steroides (0.5 mmol in 10 mL Methanol) wurde während 24h im Durchfluß zur Reaktion gebracht. Der Reaktor wurde mit Methanol gespült (10 mL) und das Lösungsmittel anschließend im Vakuum entfernt. Ausbeute an desilyliertem Steroid: > 90 % zusammen mit Spuren an Startmaterial.

### 5. Reduktive Aminierung eines Steroids

Die Chlorid-Form des Mikroreaktors wurde nacheinander behandelt mit NaOH (10 mmol in 10 mL Wasser), demineralisiertem Wasser (10 mL), Natriumborhydrid (1g in 10 mL Wasser), demineralisiertem Wasser (10 mL) und Methanol (10 mL).
Eine Lösung des Steroidketons und Benzylamine (jeweils 0.5 mmol in 10 mL Methanol) wurde über 12h bei Raumtemperatur bis zum vollständigen Umsatz durch den Reaktor circuliert. Der Reaktor wurde mit Methanol (10 mL) gewaschen und das Lösungsmittel im Vakuum entfernt. Ausbeute: > 85 %.

### 6. Generelle Vorschrift zur Synthese von Aziden

Die Chlorid-Form des Mikroreaktors wurde nacheinander behandelt mit NaOH (10 mmol in 10 mL Wasser), demineralisiertem Wasser (10 mL), Natriumazid (1g in 10 mL Wasser), demineralisiertem Wasser (10 mL), Methanol (10 mL) und Benzol (10mL).
Eine Lösung von Benzylbromid (0.5 mmol in 10 mL Benzol) wurde bei 70 °C über 12h bis zur vollständigen Reaktion durch den Reaktor gepumpt. Nach dem Entfernen des Lösungsmittels wurde Benzylazid in einer Ausbeute > 99 % erhalten.

### 7. Synthese von Benzylcyanid

In Analogie zu 6., allerdings unter Verwendung von Natriumazid erhielt man nach 12h Reaktion im Durchfluß bei 70°C Benzylcycanid in > 99 % Ausbeute.

### 8. Synthese von Octylthiocyanat

Unter Verwendung von Natriumthiocyanat (1g in 10 mL Wasser) erhielt man analog zu 6 aus Octyliodid (0.5 mmol in 10 mL Benzol) nach einer Reaktionszeit von 6d bei 70°C, Spülen des Reaktors mit Benzol (10 mL) und Entfernen des Lösungsmittels Octylthiocyanat in einer Ausbeute > 85 % und 15% Octyliodid.

Nach erfolgter Synthese lässt sich die Reaktandensäule wieder regenerieren. Hierzu wird sie zunächst wieder mit Chloridionen beladen, indem man eine verdünnte Salzsäure hindurchleitet, welche die an der Feststoffphase gebundenen Nebenprodukte der organischen Synthese verdrängt. Nach Spülen mit Wasser kann der Feststoffphasenreaktand wieder mit Borhydridionen beladen werden

Nach erfolgter Regenerierung kann z.B. die Lösung eines anderen Ketons oder eines anderen durch Borhydridionen zu reduzierenden Stoffes durch den porösen Polymer/Träger-Feststoffphasenreaktanden gepumpt werden und ein weiterer Stoff einer Substanzbibliothek erzeugt werden. Durch diese Technik gelingt es in kurzer Zeit umfangreiche Substanzbibliotheken mit potentiell neuen Wirkstoffen bzw. Bausteinen für deren Synthese herzustellen. Selbstverständlich ist die kombinierte Verschaltung mehrerer durchströmter Bauteile mit porösen Polymer/Träger-Feststoffphasenreaktanden zu einem Syntheseroboter, der auch mehrstufige Synthesen ausführen kann, möglich.

Die hier beschriebenen porösen Polymer/Träger-Feststoffphasenreaktanden und deren Verwendung stellen nur eine Auswahl dar, um die prinzipiellen Möglichkeiten aufzuzeigen. Eine große Zahl von Funktionalisierungsreaktionen durchgeführt an handelsüblichen reinen Polymeren sind nach dem Stand der Technik bekannt. Diese lassen sich auf die hier beschriebenen Polymer/Träger-Feststoffphasenreaktanden übertragen und erlauben dem Fachmann das Anwendungspotential erheblich zu erweitern.

Zum Beispiel lässt sich der Polymer/Träger-Feststoffphasenreaktand, der vernetztes Vinylbenzylchlorid im Träger enthält, auch in der Merrifield-Synthese einsetzen. Hierdurch steht eine Möglichkeit zur Verfügung, die es erlaubt auch diese Synthese mit hohen Durchflüssen zu betreiben und damit zeitlich zu verkürzen.
Die Einlagerung der Polymere in die Trägermaterialien erfolgt nach dem hier beschriebenen Verfahren durch eine Fällungspolymerisation. Auch andere Methoden (Emulsionspolymerisation, Sol-Gel-Technik, Polymerisation unter Verwendung überkritischer Lösemittel) können geeignet sein feinteilige feste Polymerphasen im Porenraum poröser Träger zu erzeugen und somit für die Herstellung der hier vorgestellten Polymer/Träger-Feststoffphasenreaktanden eingesetzt werden.

Die Strukturen von aktiven Polymeren in porösen Polymer/Träger-Feststoffphasenreaktanden sind mit den Strukturformeln Nr. 1 bis Nr. 113 in den Tabelle 1 bis 7 angegeben. In diesen Abbildungen bedeutet der schattierte Kreis das Rückgrat der vernetzten Polymerkette.

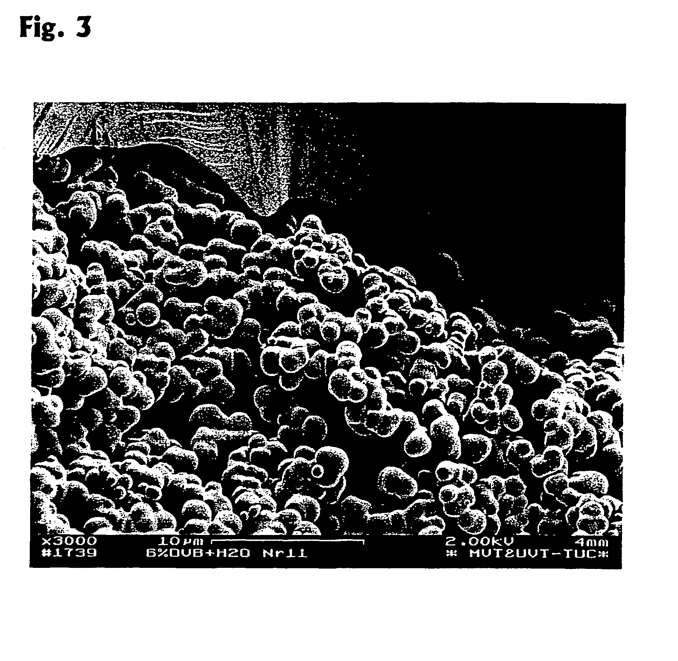

In Abbildung 3 ist eine rasterelektronenmikroskopische Aufnahme eines Querschnittes durch die hier beschriebenen porösen Polymer/Träger-Feststoffphasenreaktanden dargestellt. Im Porenraum sind deutlich die miteinander verbundenen Polymerteilchen zu sehen und die zwischen den Teilchen vorhandenen großen Poren, die eine gute konvektive Durchströmung der Feststoffphasenreaktanden erlauben. Deutlich sind die untereinander verbundenen kugelförmigen Polymerteilchen eines Feststoffphasenreaktanden zu sehen. Die Durchmesser der untereinander verbundenen Polymerteilchen liegen im Mikrometerbereich, die Durchmesser der Poren zwischen diesen Teilchen liegen ebenfalls im Mikrometerbereich.

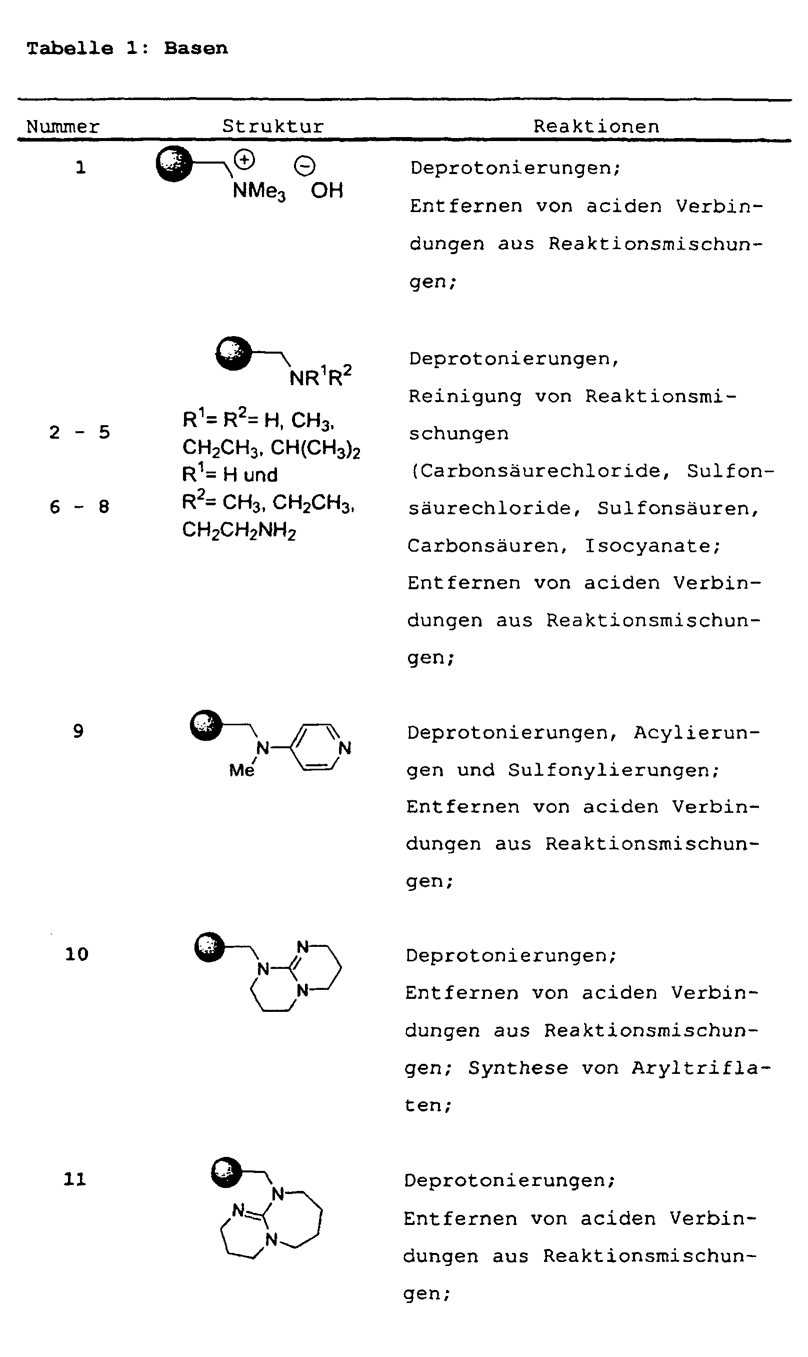

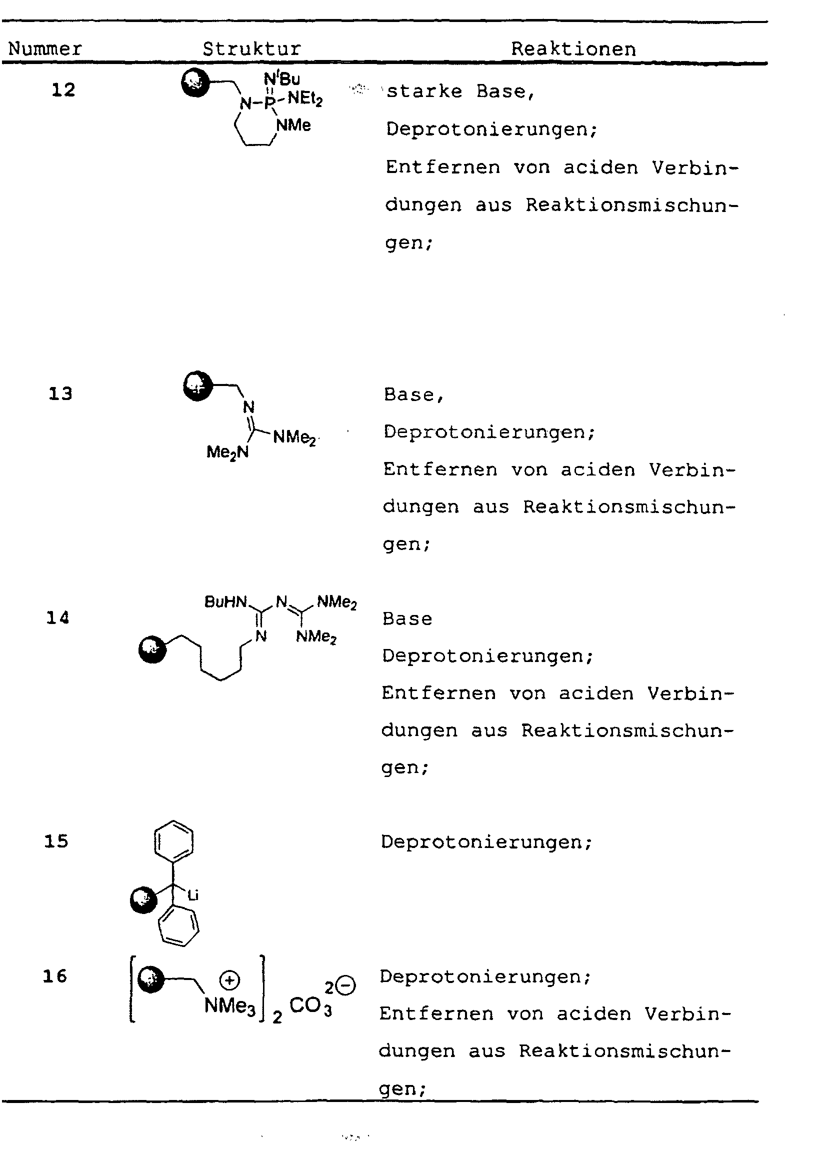

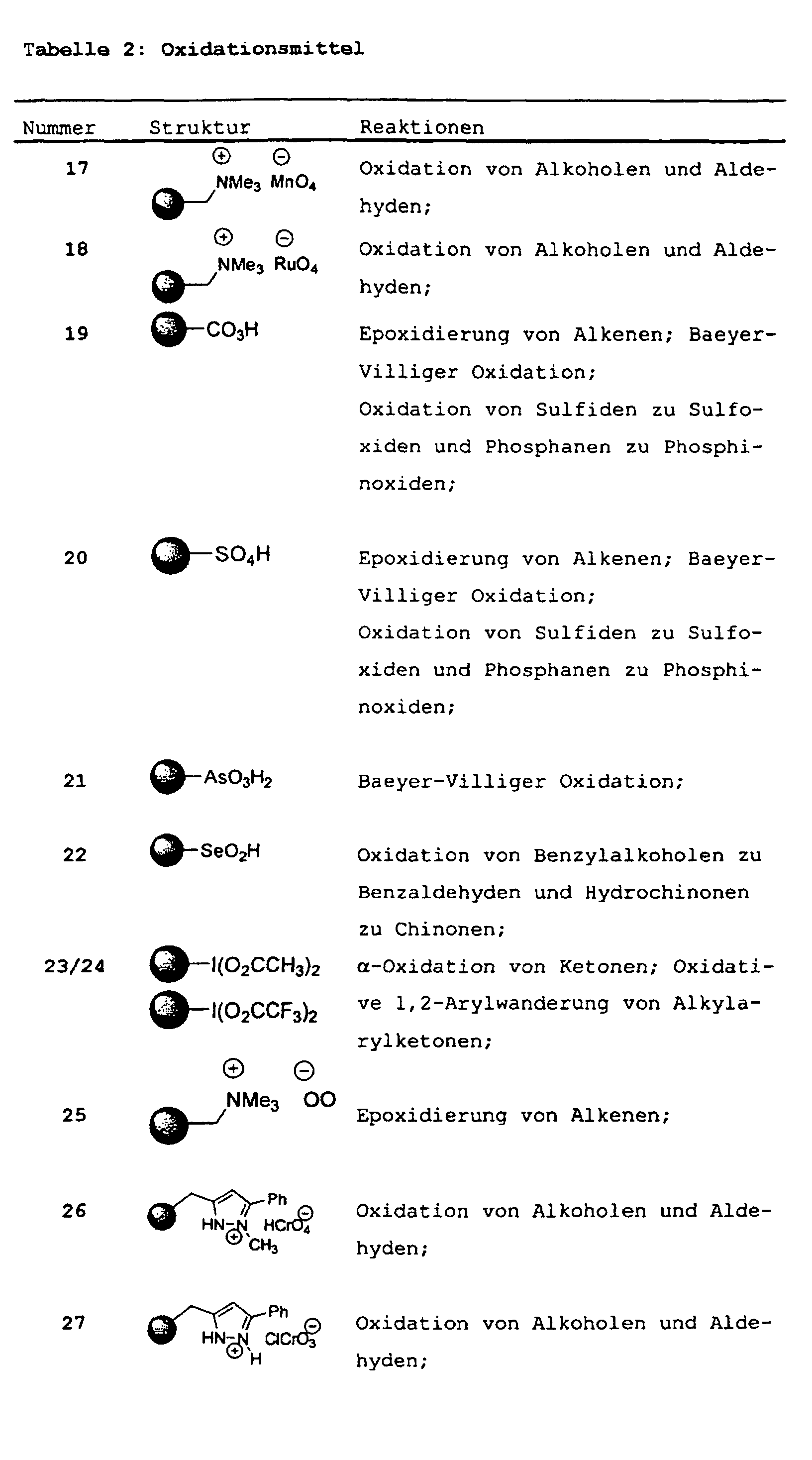

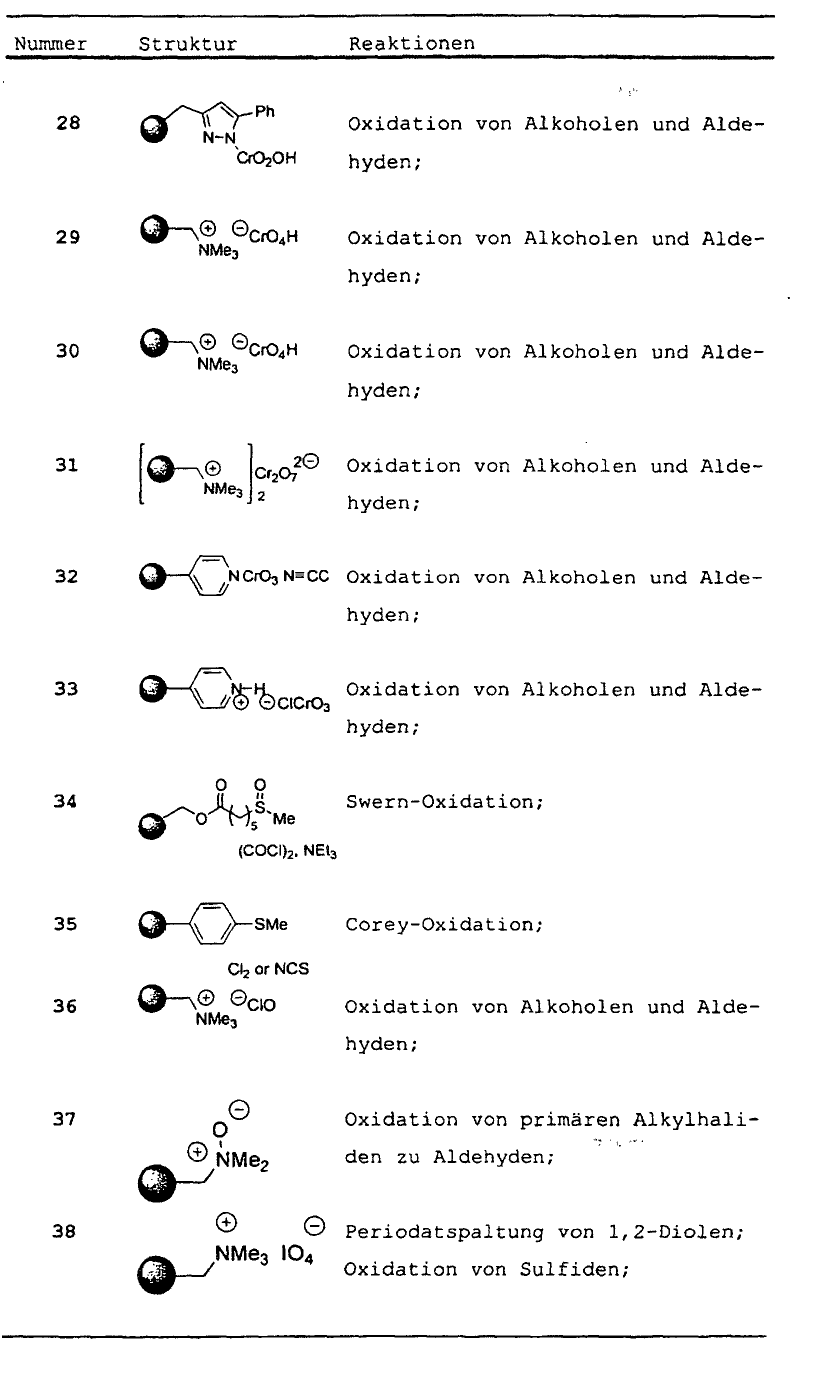

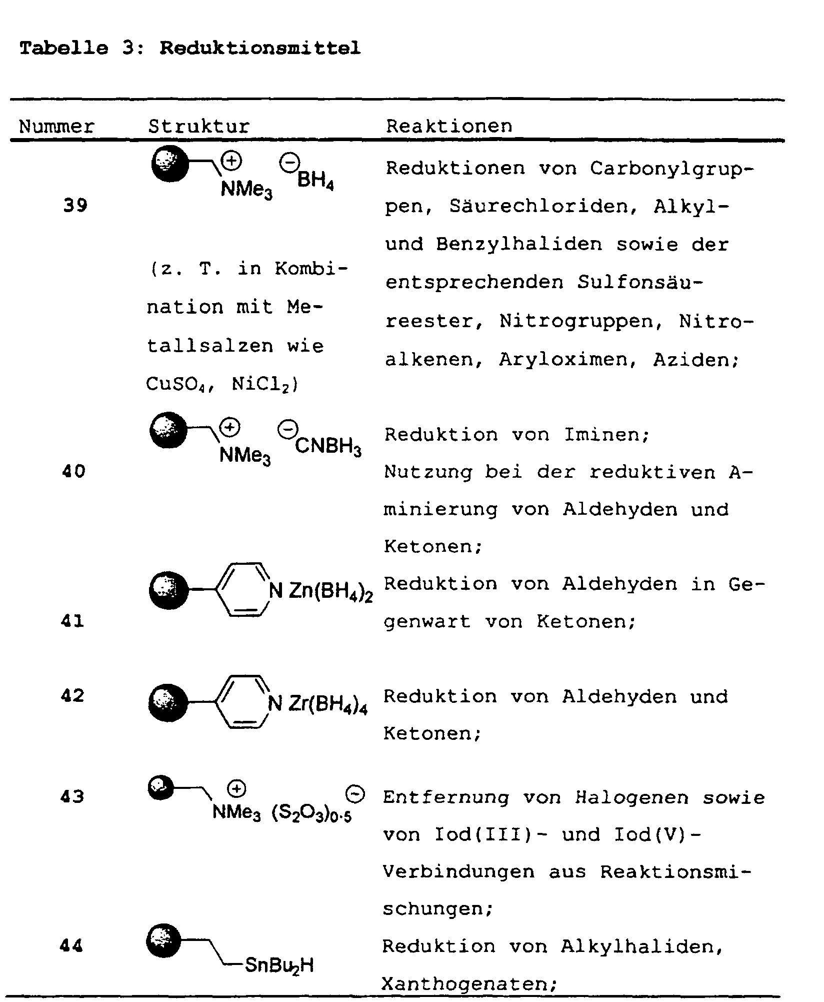

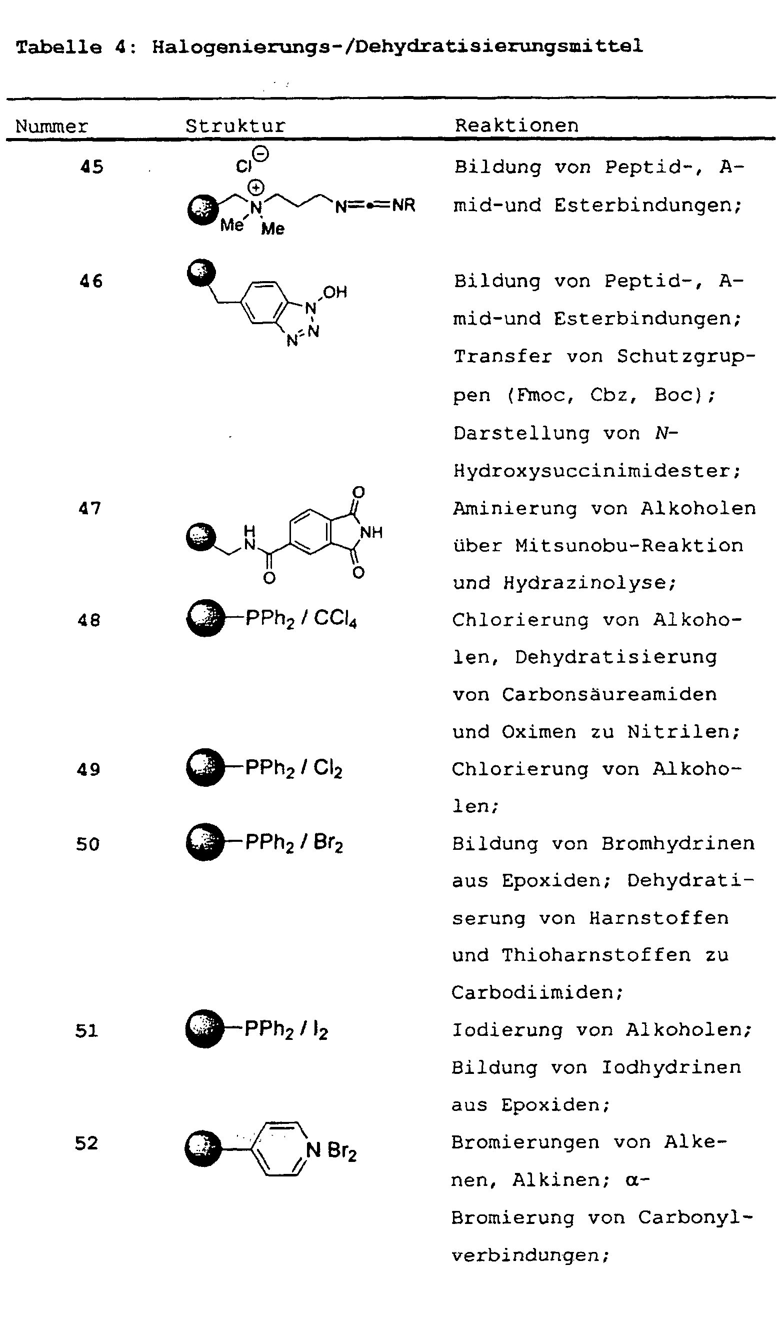

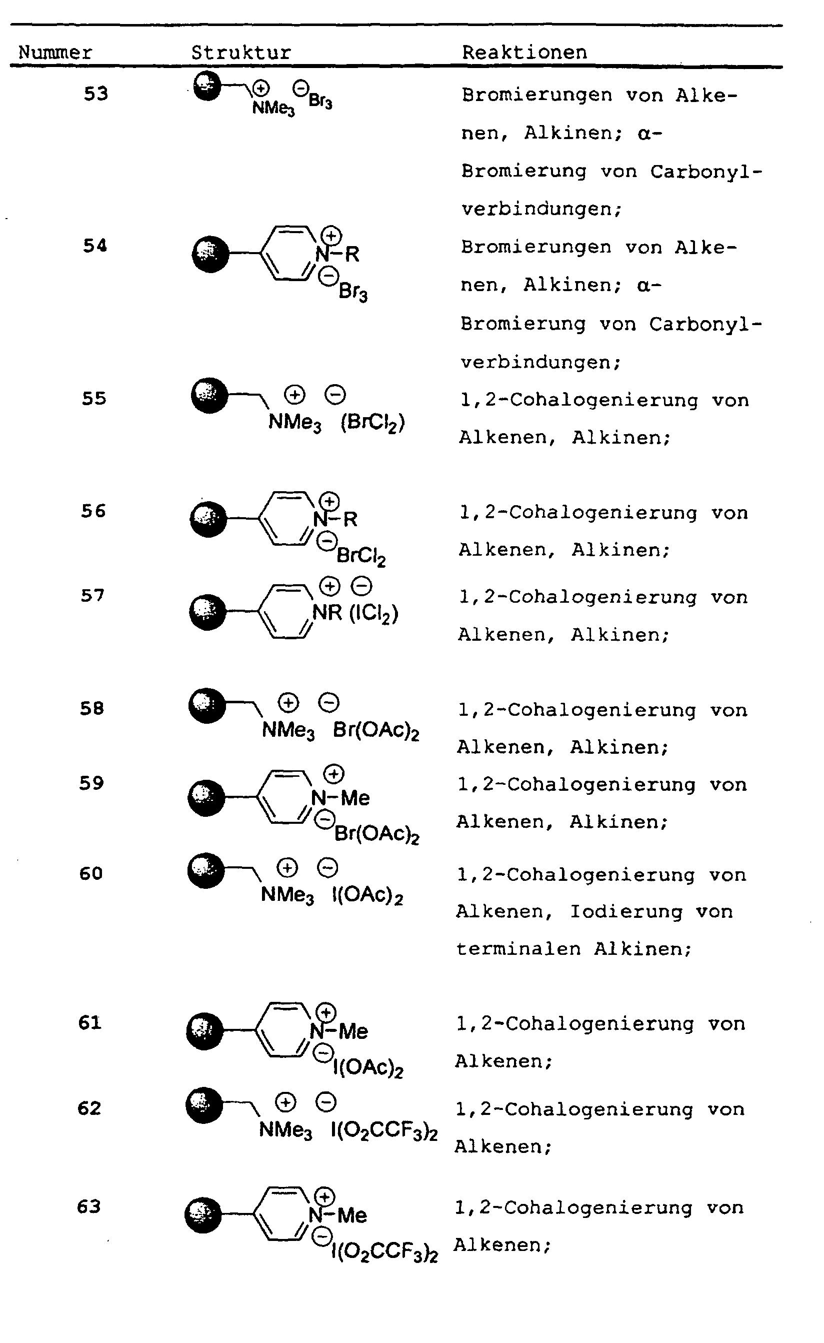

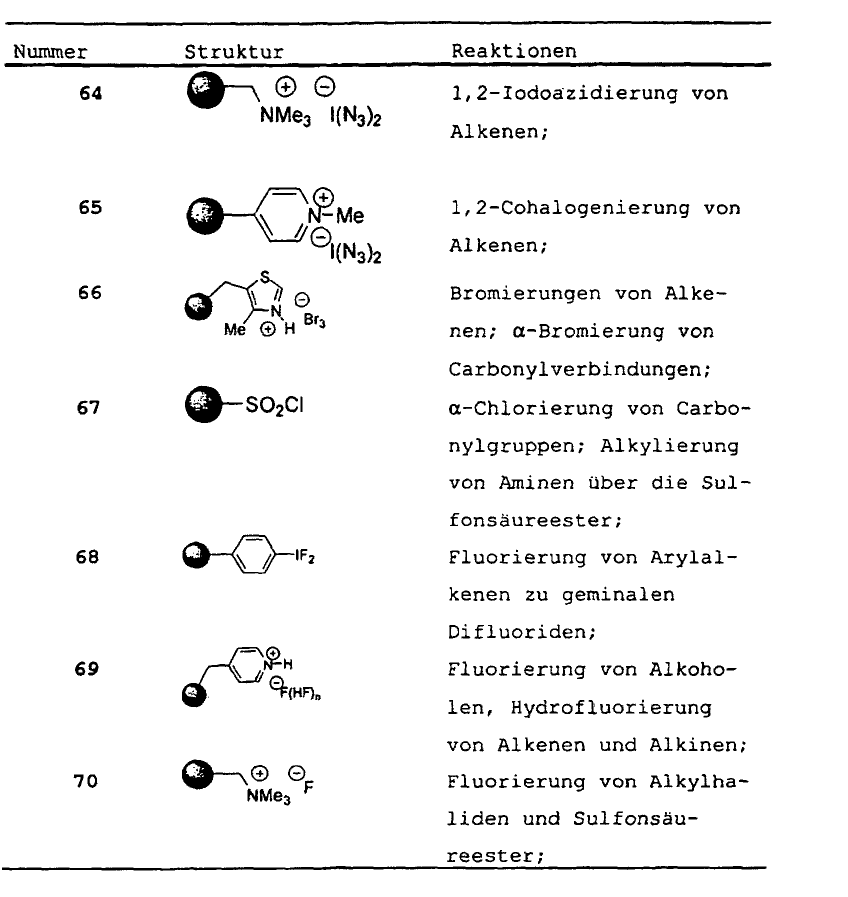

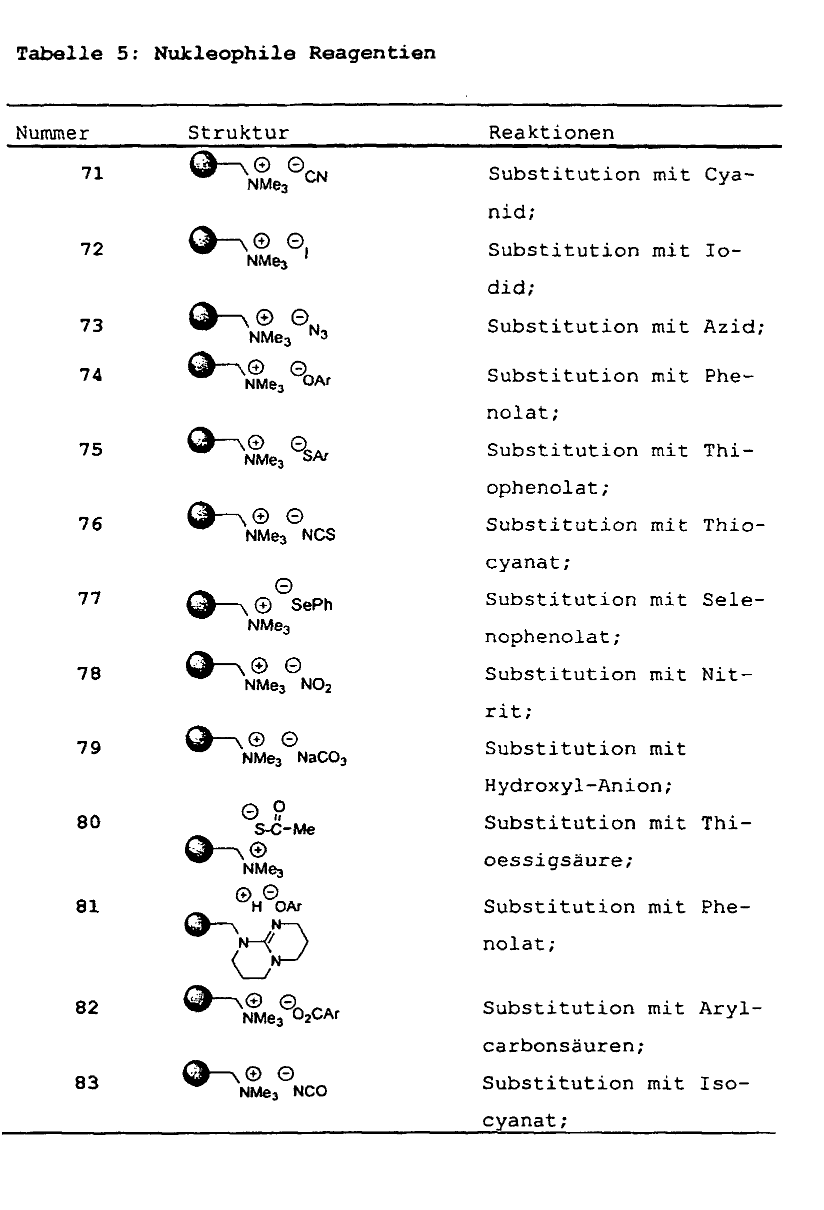

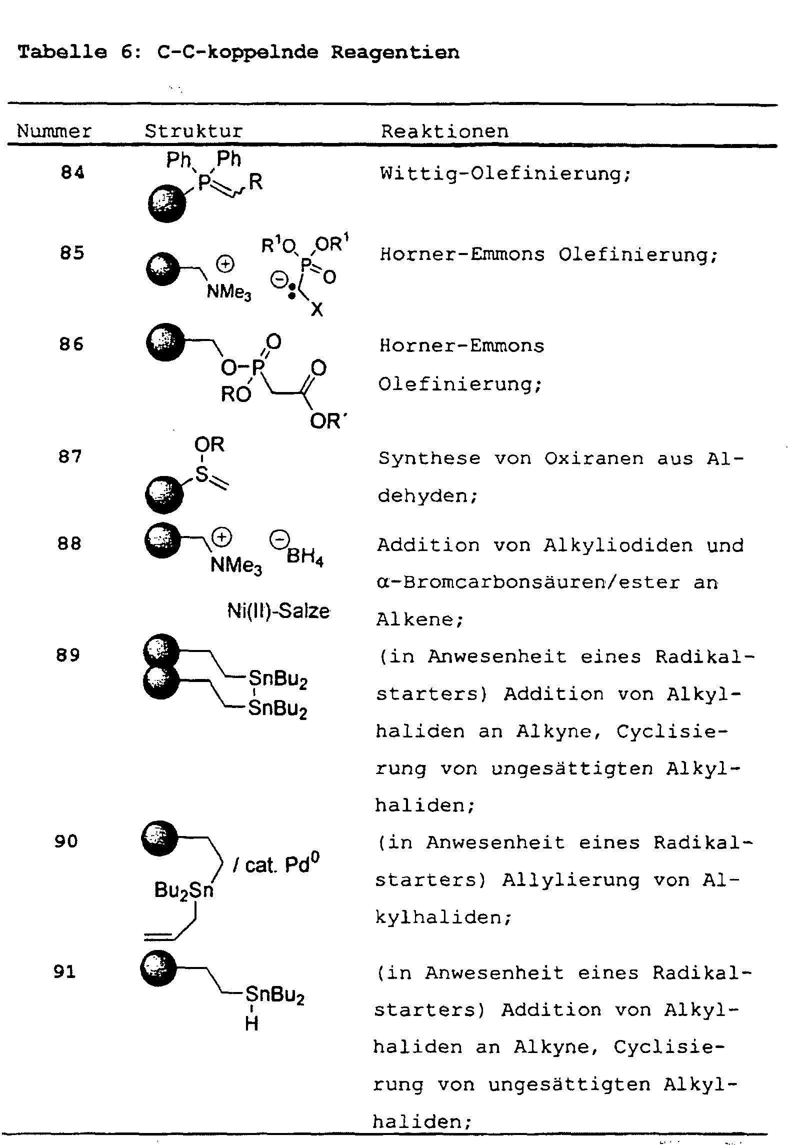

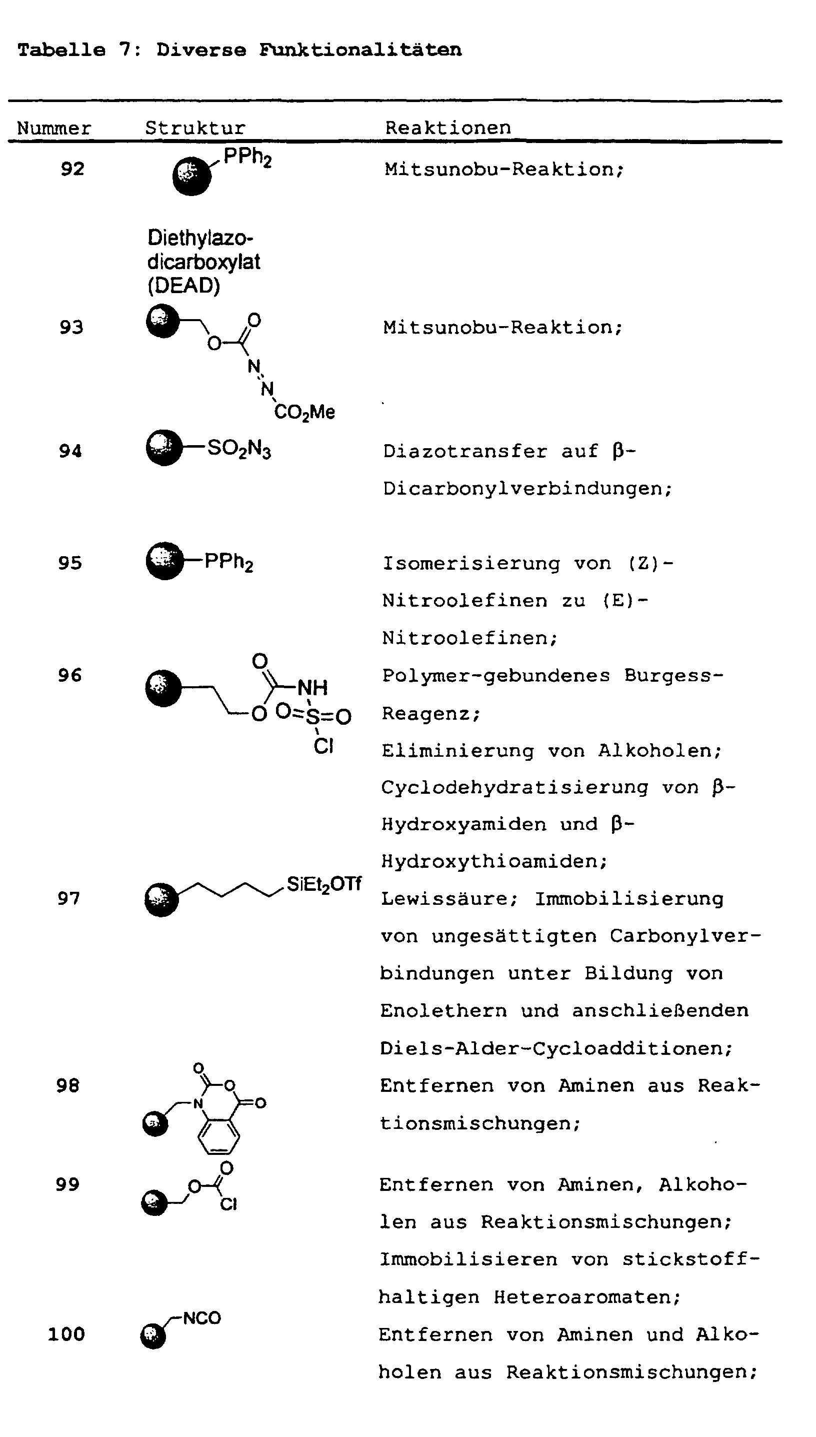

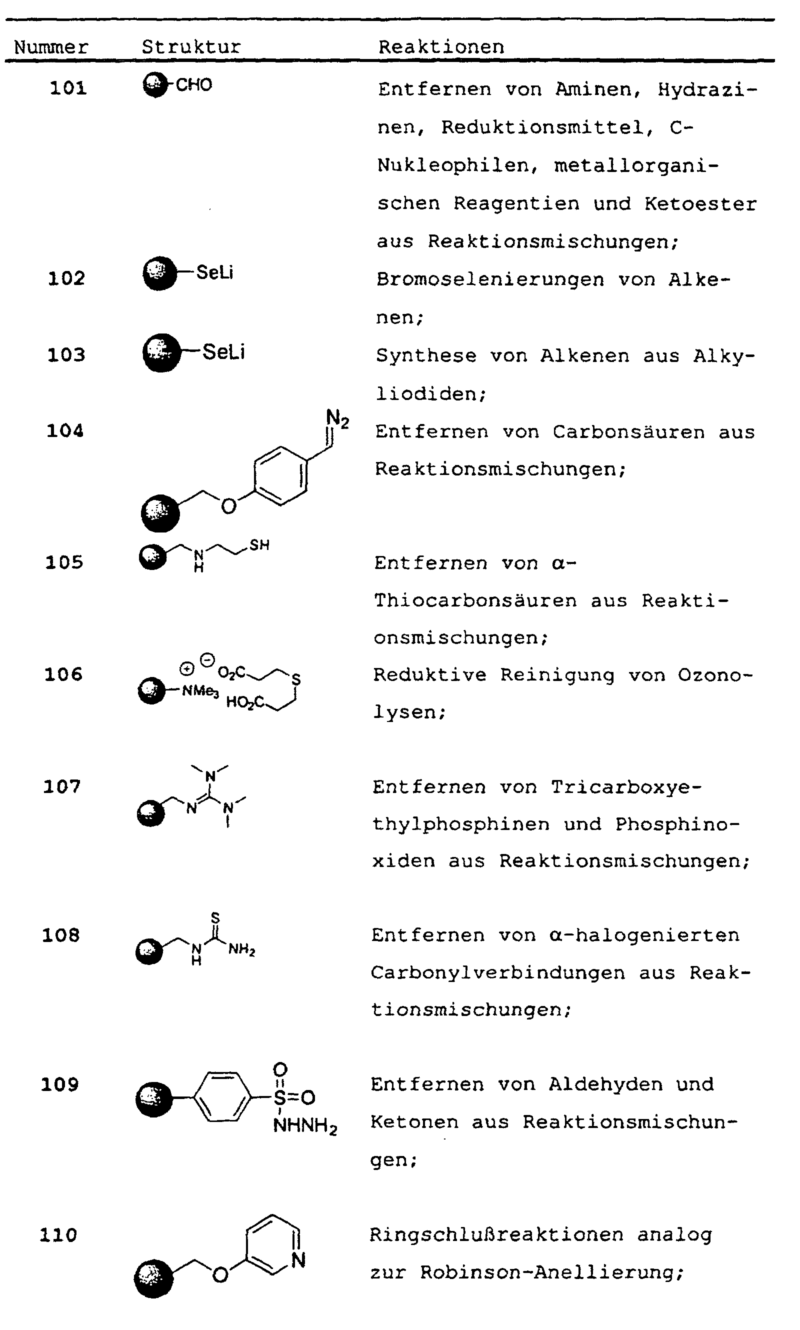

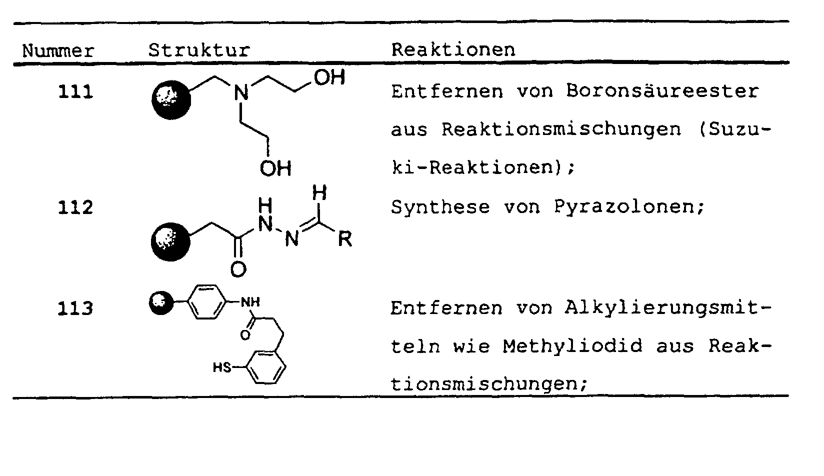

### Bezugszeichenliste

- 1 =: Vorratsbehälter
- 2 =: Pumpe
- 3, 8 =: Absperrhahn
- 4, 11 =: Rückschlagventil
- 5 =: Poröser Polymer/Träger-Feststoffphasenreaktand
- 6 =: Dosierventil
- 7 =: Auslass für Produkte
- 9 =: Behälter
- 10 =: Kreislaufpumpe
- 12 =: Leitung

## Patentansprüche

1. Polymer/Träger-Feststoffphasenreaktanden, **dadurch gekennzeichnet, dass** im Porenraum eines porösen Trägermaterials mit Porendurchmessern von 0,1 bis 2000 µm ein feinteiliges Polymer aus untereinander verbundenen Teilchen mit Durchmessern von 0,001 bis 50 µm vorliegt, an welchem organische Reaktanden gebunden sind.

2. Polymer/Träger-Feststoffphasenreaktanden nach Anspruch 1, **dadurch gekennzeichnet, dass** der Porendurchmesser des porösen Trägermaterials 10 bis 500 µm beträgt.

3. Polymer/Träger-Feststoffphasenreaktanden nach Anspruch 1, **dadurch gekennzeichnet, dass** der Teilchendurchmesser 1 bis 10 µm beträgt.

4. Polymer/Träger-Feststoffphasenreaktanden nach den vorangehenden Ansprüchen, **dadurch gekennzeichnet, dass** diese die Form von geordneten Packungen, Schüttgütern, Rohren, Platten und Stäben besitzen.

5. Polymer/Träger-Feststoffphasenreaktanden nach Anspruch 4, **dadurch gekennzeichnet, dass** diese Komponenten von Mikrotitrierfeldern sind.

6. Polymer/Träger-Feststoffphasenreaktanden nach Anspruch 4, **dadurch gekennzeichnet, dass** die Schüttgüter Kugeln, Raschig-Ringe oder Sattelkörper sind.

7. Polymer/Träger-Feststoffphasenreaktanden nach einem der vorangehenden Ansprüche, **dadurch gekennzeichnet, dass** als Polymere vernetzte Polystyrole, Polyhalogenstyrole, Polyacrylsäuren, Polyacrylsäureester, Polyacrylamide, Polyacrylnitrile, Polyvinylpyridine, Polyvinylcarbazole, Polyvinylbenzylchloride, Polyvinylaniline, Polyvinylbenzaldehyde, Poly-N-Vinylcaprolactame, Poly-4-Vinyl-1-cyclohexen-1,2-epoxide, Poly-3-Dimethylaminoacrylonitrile und Poly-N,N-Dimethylvinylbenzylamine entweder allein oder als Copolymere in den porösen Trägermaterialien vorhanden sind.

8. Polymer/Träger-Feststoffphasenreaktanden nach Anspruch 7, **dadurch gekennzeichnet, dass** als Polyhalogenstyrole Poly-4-Bromstyrol, Poly-2-Bromstyrol, Polyvinylbenzylbromid und Polyvinylbenzylchlorid verwendet werden.

9. Polymer/Träger-Feststoffphasenreaktanden nach Anspruch 7, **dadurch gekennzeichnet, dass** die Polymere mit Divinylbenzol vernetzt sind.

10. Polymer/Träger-Feststoffphasenreaktanden nach einem der Ansprüche 7 bis 9, **dadurch gekennzeichnet, dass** der Vernetzungsgrad 2 bis 55 % beträgt.

11. Polymer/Träger-Feststoffphasenreaktanden nach einem der vorangehenden Ansprüche, **dadurch gekennzeichnet, dass** die eingelagerten Polymere Ankergruppen für organische Reaktanden besitzen, nämlich Aminogruppen, quaternäre Aminogruppen, Halogengruppen, Chlormethylgruppen, Sulfochloridgruppen, Sulfonsäuregruppen, Aldehydgruppen, Lactamgruppen, Epoxidgruppen, Hydroxygruppen, Carbonsäuregruppen und Carbonsäurehalogenidgruppen.

12. Polymer/Träger-Feststoffphasenreaktanden nach einem der vorangehenden Ansprüche, **dadurch gekennzeichnet, dass** die im Porenraum des Trägermaterial vorhandenen Polymere an sich bekannte organische Reaktanden tragen.

13. Polymer/Träger-Feststoffphasenreaktanden nach einem der vorangehenden Ansprüche, **dadurch gekennzeichnet, dass** die porösen Trägermaterialien Gläser, Keramiken, Glaskeramiken, Polymere, Metalle, Legierungen, Gesteine, Koks oder Kohlen oder andere poröse Stoffe sind.

14. Polymer/Träger-Feststoffphasenreaktanden nach Anspruch 13, **dadurch gekennzeichnet, dass** die Gesteine Bimsstein und Silikate sind.

15. Verfahren zur Herstellung von Polymer/Träger-Feststoffphasenreaktanden nach einem der vorangehenden Ansprüche, **dadurch gekennzeichnet, dass** man ein vernetztes Polymer in das Trägermaterial einlagert und dieses Polymer durch eine nachfolgende Behandlung mit weiteren Monomeren funktionalisiert, wobei das Polymer gegebenenfalls Ankergruppen besitzt.

16. Verfahren nach Anspruch 15, **dadurch gekennzeichnet, dass** man die weiteren Monomere durch Eindiffundieren in die Polymerteilchen einbringt und diese weiterhin mit einem Vernetzer umsetzt.

17. Verfahren nach einem der Ansprüche 15 oder 16, **dadurch gekennzeichnet, dass** man an die Ankergruppen an sich bekannte organische Reaktanden bindet.

18. Verwendung eines Polymer/Träger-Feststoffphasenreaktanden nach einem der Ansprüche 1 bis 14 zur Durchführung von Additionsreaktionen, Oxidationen, Reduktionen, Epoxidierungen, Halogenierungen, Kohlenstoff-Kohlenstoff-Kupplungsreaktionen, nukleophilen Substitutionen, Dehydrierungen, Veresterungen, Veretherungen, Acylierungen, Ringöffnungen, Cyclisierungen, Isomerisierungen, elektrophilen Substitutionen, sowie von Reinigungsschritten und Mehrstufenreaktionen mittels der vorhandenen organischen Reaktanden.

19. Verwendung nach Anspruch 18, **dadurch gekennzeichnet, dass** es sich bei den Reinigungsreaktionen um "resin capture-and-release" und "scavenger" handelt.

20. Verwendung nach einem der Ansprüche 17 oder 18, **dadurch gekennzeichnet, dass** Stoffsynthese und Trennung im Polymer/Träger-Feststoffphasenreaktanden kombiniert sind.

## Claims

1. Polymer/support solid-phase reactants, hereby **characterized in that** a fine particulate polymer comprised of particles with diameters of 0.001 to 50 µm to which organic reactants are bound is present in the pore volume of a porous support material with pore diameters of 0.1 to 2000 µm.

2. The polymer/support solid-phase reactant according to claim 1, further **characterized in that** the pore diameter of the porous support material amounts to 10 to 500 µm.

3. The polymer/support solid-phase reactants according to claim 1, further **characterized in that** the particle diameter amounts to 1 to 10 µm.

4. The polymer/support solid-phase reactant according to the preceding claims, further **characterized in that** these have the form of ordered packings, bulk materials, tubes, plates and rods.

5. The polymer/support solid-phase reactants according to claim 4, further **characterized in that** these are components of microtitrating arrays.

6. The polymer/support solid-phase reactants according to claim 4, further **characterized in that** the bulk materials are spheres, Raschig rings or saddle-shaped pieces.

7. The polymer/support solid-phase reactants according to one of the preceding claims, further **characterized in that** cross-linked polystyrenes, polyhalostyrenes, polyacrylic acids, polyacrylic acid esters, polyacrylamides, polyacrylonitriles, polyvinylpyridines, polyvinyl carbazoles, polyvinylbenzyl chlorides, polyvinyl anilines, polyvinylbenzaldehydes, poly-N-vinyl caprolactams, poly-4-vinyl-1-cyclohexene 1,2-epoxides, poly-3-dimethylaminoacrylonitriles and poly-N,N-dimethylvinylbenzylamines are present in the porous support materials as polymers, either alone or as copolymers.

8. The polymer/support solid-phase reactants according to claim 7, further **characterized in that** poly-4-bromostyrene, poly-2-bromostyrene, polyvinylbenzyl bromide and polyvinylbenzyl chloride are used as polyhalostyrenes.

9. The polymer/support solid-phase reactants according to claim 7, further **characterized in that** the polymers are cross-linked with divinylbenzene.

10. The polymer/support solid-phase reactants according to one of claims 7 to 9, further **characterized in that** the degree of cross-linking amounts to 2 to 55%.

11. The polymer/support solid-phase reactants according to one of the preceding claims, further **characterized in that** the embedded polymers possess anchoring groups for organic reactants, i.e., amino groups, quaternary amino groups, halogen groups, chloromethyl groups, sulfochloride groups, sulfonic acid groups, aldehyde groups, lactam groups, epoxide groups, hydroxy groups, carboxylic acid groups and carboxylic acid halide groups.

12. The polymer/support solid-phase reactants according to one of the preceding claims, further **characterized in that** the polymers present in the pore volume of the support material support organic reactants which are known in the art.

13. The polymer/support solid-phase reactants according to one of the preceding claims, further **characterized in that** the porous support materials are glasses, ceramics, glass ceramics, polymers, metals, alloys, stones, coke or coal or other porous substances.

14. The polymer/support solid-phase reactants according to claim 13, further **characterized in that** the stones are pumice stone and silicates.

15. A method for the production of polymer/support solid-phase reactants according to one of the preceding claims, hereby **characterized in that** a cross-linked polymer is embedded in the support material and this polymer is functionalized by a subsequent treatment with other monomers, whereby the polymer possesses anchoring groups, if necessary.

16. The method according to claim 15, further **characterized in that** the other monomers are introduced by diffusing into the polymer particles and then these are reacted with a cross-linker.

17. The method according to one of claims 15 or 16, further **characterized in that** organic reactants that are known in the art are bound to the anchoring groups.

18. Use of a polymer/support solid-phase reactant according to one of claims 1 to 14 for conducting addition reactions, oxidations, reductions, epoxidations, halogenations, carbon-carbon coupling reactions, nucleophilic substitutions, dehydrogenations [dehydrations], esterifications, etherifications, acylations, ring openings, cyclizations, isomerizations, electrophilic substitutions, as well as purification steps and multi-stage reactions by means of the organic reactants present.

19. The use according to claim 18, further **characterized in that** the purification reactions involve "resin capture-and-release" and "scavenger" steps.

20. The use according to one of claims 17 or 18, further **characterized in that** the substance is synthesized and separated in a combined manner in the polymer/support solid-phase reactant.

## Revendications

1. Réactants en phase solide polymère/support, **caractérisés en ce que**, dans l'espace poreux d'un matériau support poreux avec diamètres de pores de 0,1 à 2000 µm, il existe un polymère à particules fines constitué de particules liées les unes aux autres ayant des diamètres de 0,001 à 50 µm, auquel des réactants organiques sont liés.

2. Réactants en phase solide polymère/support selon la revendication 1, **caractérisés en ce que** le diamètre des pores du matériau support poreux est de 10 à 500 µm.

3. Réactants en phase solide polymère/support selon la revendication 1, **caractérisés en ce que** le diamètre des particules est de 1 à 10 µm.

4. Réactants en phase solide polymère/support selon les revendications précédentes, **caractérisés en ce que** ceux-ci possèdent la forme de conditionnements, de produits en vrac, de tubes, de plaques et de barres ordonnés.

5. Réactants en phase solide polymère/support selon la revendication 4, **caractérisés en ce que** ceux-ci sont les composants de zones microtitrées.

6. Réactants en phase solide polymère/support selon la revendication 4, **caractérisés en ce que** les produits en vrac sont des billes, des anneaux de Raschig ou des corps en forme de selle.

7. Réactants en phase solide polymère/support selon l'une des revendications précédentes, **caractérisés en ce que**, en tant que polymères, des polystyrènes réticulés, styrènes polyhalogénés, acides polyacryliques, esters d'acides polyacryliques, polyacrylamides, polyacrylnitriles, polyvinylpyridines, polyvinylcarbazols, chlorures de polyvinylbenzyle, polyvinylanilines, polyvinylbenzaldéhydes, poly-N-vinylcaprolactames, poly-4-vinyl-1-cyclohexen-1,2-époxydes, poly-3-diméthylaminoacrylonitriles et poly-N,N-diméthylvinylbenzylamines sont présents, soit seuls soit en tant que copolymères dans les matériaux supports poreux.

8. Réactants en phase solide polymère/support selon la revendication 7, **caractérisés en ce que**, en tant que styrènes polyhalogénés, du poly-4-bromstyrène, du poly-2-bromstyrène, du bromure de polyvinylbenzyle et du chlorure de polyvinylbenzyle sont utilisés.

9. Réactants en phase solide polymère/support selon la revendication 7, **caractérisés en ce que** les polymères sont réticulés avec du divinylbenzène.

10. Réactants en phase solide polymère/support selon l'une des revendications 7 à 9, **caractérisés en ce que** le degré de réticulation est de 2 à 55 %.

11. Réactants en phase solide polymère/support selon l'une des revendications précédentes, **caractérisés en ce que** les polymères emmagasinés possèdent des groupes de fixation pour des réactants organiques, c'est-à-dire des groupes amino, des groupes amino quaternaires, des groupes halogènes, des groupes chlorure de méthyle, des groupes sulfochlorure, des groupes acide sulfonique, des groupes aldéhydes, des groupes lactames, des groupes époxydes, des groupes hydroxy, des groupes acide carboxylique et des groupes halogénures d'acide carboxylique.

12. Réactants en phase solide polymère/support selon l'une des revendications précédentes, **caractérisés en ce que** les polymères présents dans l'espace poreux du matériau support supportent des réactants organiques connus.

13. Réactants en phase solide polymère/support selon l'une des revendications précédentes, **caractérisés en ce que** les matériaux supports poreux sont des verres, céramiques, vitrocéramiques, polymères, métaux, alliages, roches, cokes ou charbons ou d'autres substances poreuses.

14. Réactants en phase solide polymère/support selon la revendication 13, **caractérisés en ce que** les roches sont de la pierre ponce et des silicates.

15. Procédé de production de réactants en phase solide polymère/support selon l'une des revendications précédentes, **caractérisé en ce que** l'on emmagasine un polymère réticulé dans le matériau support et fonctionnalise ce polymère par un traitement consécutif avec d'autres monomères, de sorte que le polymère possède le cas échéant des groupes de fixation.

16. Procédé selon la revendication 15, **caractérisé en ce que** l'on apporte les autres monomères, par diffusion à l'intérieur, dans les particules de polymère et l'on met ensuite ceux-ci en réaction avec un réticulant.

17. Procédé selon l'une des revendications 15 ou 16, **caractérisé en ce que** l'on lie aux groupes de fixation des réactants organiques connus.

18. Utilisation d'un réactant en phase solide polymère/support selon l'une des revendications 1 à 14 pour la réalisation de réactions d'addition, d'oxydations, de réductions, d'époxydations, d'halogénations, de réactions de couplage carbone-carbone, de substitutions nucléophiles, de déshydrogénations, d'estérifications, d'éthérifications, d'acylations, d'ouvertures de cycles, de cyclisations, d'isomérisations, de substitutions électrophiles, ainsi que de mesures de purification et de réactions en plusieurs étapes, au moyen des réactants organiques présents.

19. Utilisation selon la revendication 18, **caractérisée en ce que** les réactions de purification consistent en "capture et libération de résines" et "fixateur".

20. Utilisation selon l'une des revendications 17 ou 18, **caractérisée en ce que** la synthèse des substances et la séparation sont combinées dans les réactants en phase solide polymère/support.
